(19) Europäisches Patentamt
European Patent Office
Office européen des brevets

(11) **EP 4 401 084 A2**

(12) **EUROPEAN PATENT APPLICATION**

(43) Date of publication:
**17.07.2024 Bulletin 2024/29**

(21) Application number: **23218019.0**

(22) Date of filing: **19.12.2023**

(51) International Patent Classification (IPC):
**G16H 20/17** (2018.01)   **G16H 40/63** (2018.01)
**A61M 5/14** (2006.01)   **A61M 5/142** (2006.01)
**G10L 13/00** (2006.01)   **G10L 15/00** (2013.01)

(52) Cooperative Patent Classification (CPC):
**A61M 5/14; A61M 5/14244; A61M 5/14248;**
**G16H 20/17; G16H 40/63;** G06F 16/3344;
G06F 40/56; G10L 13/00; G10L 15/00

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **19.12.2022 US 202263476077 P**

(71) Applicant: **Insulet Corporation**
**Acton MA 01720 (US)**

(72) Inventor: **NARAYANASWAMI, Rangarajan**
**Weston (US)**

(74) Representative: **Peterreins Schley**
**Patent- und Rechtsanwälte PartG mbB**
**Hermann-Sack-Straße 3**
**80331 München (DE)**

(54) **AUDIO/TACTILE INPUT-OUTPUT AND AUDIO CONTROL FOR AUTOMATED INSULIN DELIVERY APPLICATIONS**

(57)   Disclosed are techniques, devices and systems that implement and utilize a voice control application and sound chip circuitry to control operation of a wearable drug delivery device. In addition, a wearable drug delivery device equipped with the sound chip circuitry may be operable to play audio clips that may include content for assisting a user with an initial set up of the wearable drug delivery device, providing messages of encouragement, lullabies for pediatric users, and the like. In addition, the voice control application enables users to control delivery of liquid drugs via a wearable drug delivery device hands-free using voice commands and voice confirmations of actions to be taken by an automated insulin delivery application.

FIG. 1

**Description**

BACKGROUND

A person with diabetes may use an automated insulin delivery (AID) system to monitor and deliver insulin according to an insulin treatment plan. Interactions with the AID system are commonly through a controller that requires a user to read prompts requesting information, understand what information is being requested, and enter the information via a user interface of the controller. While older children, adolescents, and adults may not have problems understanding what information is requested and entering the requested information, younger children and the elderly may have difficulty with providing the requested information. The difficulty may arise from a number of factors, such as a lack of understanding of the request, an inability to navigate the controller's user interface due to confusion, impaired visual abilities (including vision impairments due to diabetic retinopathy), and/or a lack of physical dexterity, and the like.

In other instances, the interaction with the user interface is simply inconvenient or perhaps impractical, the device with the user interface is lost or located away from the person utilizing the AID system, or the person utilizing the AID system is engaged with another activity, such as driving, or exercising (e.g., bike riding, rowing, paddleboarding, running, and the like), or using their hands, such as by carrying groceries, luggage, or the like.

It would be beneficial if parts of an AID system, such as a wearable drug delivery device, personal diabetes management device, or a portable device, were available to receive, store and respond to audio inputs, such as voice commands, messages or comments, specific audio signals, or the like, to take different actions related to a user's insulin treatment plan.

In a related situation, it has been shown that users of AID system may suffer some level of emotional distress when implementing an AID system, and empathetic and supportive words or calming music may help ease the emotional distress. It would be further helpful to have parts of the AID system configured to output and receive audio messages, announcements or AID-related device usage feedback or the like to help make the implementation less emotionally stressful.

SUMMARY

Disclosed is a wearable drug delivery device including a processor, communication circuitry, sound chip circuitry, and a memory. The processor may be operable to execute programming instructions. The communication circuitry may be coupled to the processor and operable to receive and transmit information. The sound chip circuitry may include a recording memory, a microphone, a speaker, a processor, and a voice control application. The recording memory, the microphone, the speaker, and the voice control circuitry may be coupled to one another and the recording memory may be operable to store a plurality of audio clips. The memory may be coupled to the processor and operable to store the programming instructions. The sound chip circuitry may be operable to identify a specific audio clip from the plurality of audio clips stored in the recording memory, and output the specific audio clip via the speaker.

Disclosed is a wearable drug delivery device including a processor, communication circuitry, sound chip circuitry and a memory. The processor may be operable to execute programming instructions. The communication circuitry may be coupled to the processor and may be operable to receive and transmit information. The sound chip circuitry may include a microphone, a speaker, and voice control circuitry. The microphone, the speaker and the voice control circuitry may be coupled to one another. The memory may be coupled to the processor and may be operable to store the programming instructions. The sound chip circuitry may be operable to receive a voice command indicative of an insulin delivery action to be taken by an automated insulin delivery application. The sound chip circuitry may determine that the received voice command corresponds to an identified insulin delivery action to be taken by the automated insulin delivery application, and output a confirmation request. The sound chip circuitry may monitor for an input that is a confirmation response to the confirmation request; and, in response to receipt of the confirmation response, enable the insulin delivery action to be taken by the automated insulin delivery application.

A drug delivery system is also disclosed. The drug delivery system may include a processor, communication circuitry, sound chip circuitry, and a memory. The processor may be operable to execute programming instructions. The communication circuitry may be coupled to the processor and may be operable to receive and transmit information. The sound chip circuitry may include a microphone, a speaker, a vibration device, and voice control circuitry. The memory may be coupled to the processor and may be operable to store the programming instructions. The sound chip circuitry may be operable to receive a voice command indicative of an insulin delivery action to be taken by an automated insulin delivery application. The sound chip circuitry may be operable to determine that the received voice command corresponds to an identified insulin delivery action to be taken by the automated insulin delivery application, and output a confirmation request. The sound chip circuitry may also monitor for receipt of an input that is a confirmation response to the confirmation request; and in response to receipt of the confirmation response, enable the insulin delivery action to be taken by the automated insulin delivery application.

BRIEF DESCRIPTION OF THE DRAWINGS

FIG. 1 illustrates a block diagram representation of an exemplary drug delivery system that is suitable to implement the subject matter described herein.

FIG. 2 illustrates a block diagram representation of an example of AID system configured to provide audio messages according to the described examples;

FIG. 3 illustrates a block diagram representation of features and functions provided by the example wearable drug delivery device of FIG. 2;

FIG. 4 illustrates another block diagram representation of an example of AID system configured to provide audio messages according to the described examples;

FIG. 5 illustrates an example of a voice control pipeline for controlling an AID system according to the disclosed subject matter;

FIG. 6A is a flowchart of an exemplary general voice control process according to the disclosed subject matter;

FIG. 6B illustrates an exemplary voice control progression for causing an AID system to deliver a bolus for a meal with known nutritional information according to the disclosed subject matter;

FIG. 7 illustrates an example voice control progression for causing an AID system to deliver a meal bolus with correction dosage according to the disclosed subject matter;

FIG. 8 illustrates an example voice control progression for causing an AID system to deliver a correction bolus according to the disclosed subject matter;

FIG. 9 illustrates an example voice control progression for causing an AID system to deliver a quick meal bolus according to the disclosed subject matter;

FIG. 10 illustrates an example voice control progression for causing an AID system to deliver a bolus based on a meal description according to the disclosed subject matter;

FIG. 11 illustrates an example voice control progression for causing an AID system to deliver basal insulin at a reduced basal rate according to the disclosed subject matter;

FIG. 12 illustrates an example voice control progression for causing an AID system to set an activity mode of an AID algorithm according to the disclosed subject matter;

FIG. 13 illustrates an example voice control progression for causing an AID system to receive confirmation of AID algorithm action according to the disclosed subject matter;

FIG. 14 illustrates an example voice control progression for interacting with an AID system control menu according to the disclosed subject matter;

FIG. 15 illustrates an example voice control progression for causing an AID system to record annotations related the AID system according to the disclosed subject matter; and

FIG. 16 illustrates an example of evaluating annotations with respect to metrics affecting a user's insulin treatment program according to the disclosed subject matter.

DETAILED DESCRIPTION

Systems, devices, and methods in accordance with the present disclosure will now be described more fully hereinafter with reference to the accompanying drawings, where one or more embodiments are shown. The systems, devices, and methods may be embodied in many different forms and are not to be construed as being limited to the embodiments set forth herein. Instead, these embodiments are provided so the disclosure is thorough and complete, and fully conveys the scope of methods and devices to those skilled in the art. Each of the systems, devices, and methods disclosed herein provides one or more advantages over conventional systems, components, and methods.

A type of automated insulin delivery (AID) system may include an "artificial pancreas" algorithm-based system, or more generally, an artificial pancreas (AP) application. For ease of discussion, the computer programs and computer applications that implement the medication delivery algorithms or applications may be referred to herein as an "AP application." An AP application may be configured to provide automatic delivery of insulin based on an analyte sensor input.

In addition, or alternatively, while the AID application actions may have been described with reference to a closed loop algorithmic implementation, variations of the disclosed examples may be implemented to enable open loop use. The open loop implementations allow for use of different modalities of delivery of insulin such as smart pen, syringe, or the like in addition to the described wearable drug delivery device. For example, the disclosed AP application and algorithms may be operable to perform various functions related to open loop operations, such as determination of a total daily setting for a drug or combination of drugs, such as a total daily insulin setting or the like.

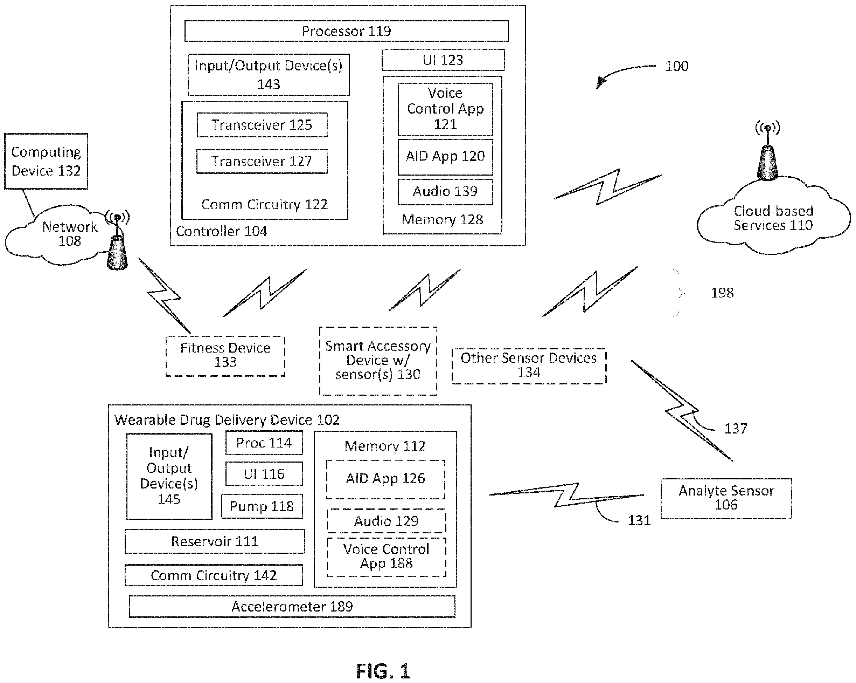

FIG. 1 illustrates a simplified block diagram of a drug delivery system 100.

In some examples, the drug delivery system 100 is suitable for delivering insulin to a user in accordance with the disclosed embodiments. The drug delivery system 100 may include a wearable drug delivery device 102, a controller 104 and an analyte sensor 106. In addition, the drug delivery system may interact with a computing device 132 via a network 108 as well as obtain or contribute to cloud-based services 110.

The wearable drug delivery device 102 may be a wearable device that is worn on the body of the user. The wearable drug delivery device 102 may be directly coupled to a user (e.g., directly attached to the skin of the user via an adhesive, or the like at various locations on the user's body, such as thigh, abdomen, or upper arm). In an example, a surface of the wearable drug delivery device 102 may include an adhesive to facilitate attachment to the skin of a user.

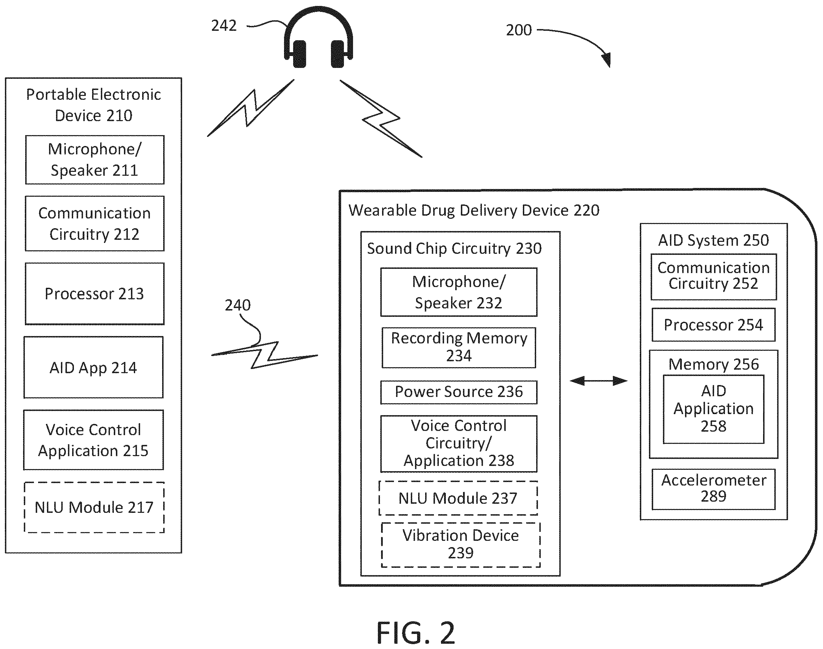

The wearable drug delivery device 102 may include a processor 114. The processor 114 may be implemented in hardware, software, or any combination thereof. The processor 114 may, for example, be a microprocessor, a logic circuit, a field programmable gate array (FPGA), an application specific integrated circuit (ASIC) or a microprocessor coupled to a memory. The processor 114 may maintain a date and time as well as be operable to perform other functions (e.g., calculations or the like). The processor 114 may be operable to execute programming instructions related to the different functions performed by the wearable drug delivery device. The programming instructions may include an AID application 126 and a voice control application 188 stored in the memory 112 that enables the processor 114 to direct operation of the wearable drug delivery device 102. The AID application 126 may control insulin delivery to the user per an AID algorithm. The memory 112 may store AID application settings for a user, such as specific factor settings, subjective insulin need parameter settings, and AID algorithm settings, such as maximum insulin delivery, insulin sensitivity settings, total daily insulin (TDI) settings and the like. The memory may also store audio data 129, such as pre-recorded audio clips, downloaded audio clips, contemporaneously recorded audio clips, and the like as described with reference to FIGs. 2-16.

The analyte sensor 106 may be operable to collect physiological condition data, such as the blood glucose measurement values and a timestamp, ketone levels, heart rate, blood oxygen levels and the like that may be shared with the wearable drug delivery device 102, the controller 104 or both. For example, the communication circuitry 142 of the wearable drug delivery device 102 may be operable to communicate with the analyte sensor 106 and the controller 104 as well as the devices 130, 133 and 134. The communication circuitry 142 may be operable to communicate via Bluetooth®, Wi-Fi, a near-field communication standard, a cellular standard, or any other wireless protocol.

The input/output device(s)145 may have one or more of a microphone, a speaker, a vibration device, a display, a push button, a touchscreen display, a tactile input surface, or the like. The input/output device(s) 145 may be coupled to the processor 114 and may include circuitry operable to generate signals based on received inputs and provide the generated signals to the processor 114. In an example, a vibration device as an input/output device 145 may be operable to generate vibrations that signal different information or operations (e.g., bolus delivery, command receipt confirmation or the like). In addition, the input/output device(s) 145 may be operable to receive signals from the processor 114 and, based on the received signals, generate outputs via a respective output device.

The wearable drug delivery device 102 may include a reservoir 111. The reservoir 111 may be operable to store drugs, medications, or therapeutic agents suitable for automated delivery, such as insulin, morphine, methadone, hormones, glucagon, glucagon-like peptide, blood pressure medicines, chemotherapy drugs, combinations of drugs, such as insulin and glucagon-like peptide, or the like. A fluid path to the user may be provided via tubing and a needle/cannula (not shown). The fluid path may, for example, include tubing coupling the wearable drug delivery device 102 to the user (e.g., via tubing coupling a needle or cannula to the reservoir 111). The wearable drug delivery device 102 may be operable based on control signals from the processor 114 to expel the drugs, medications, or therapeutic agents, such as insulin, from the reservoir 111 to deliver doses of the drugs, medications, or therapeutic agents, such as the insulin, to the user via the fluid path. For example, the processor 114 by sending control signals to the pump 118 may be operable to cause insulin to be expelled from the reservoir 111.

There may be one or more communication links 198 with one or more devices physically separated from the wearable drug delivery device 102 including, for example, a controller 104 of the user and/or a caregiver of the user and/or a sensor 106. The analyte sensor 106 may communicate with the wearable drug delivery device 102 via a wireless communication link 131 and/or may communicate with the controller 104 via a wireless communication link 137. The communication links 131, 137, and 198 may include wired or wireless communication paths operating according to any known communications protocol or standard, such as Bluetooth, Wi-Fi, a near-field communication standard, a cellular standard, or any other wireless protocol.

The wearable drug delivery device 102 may also include a user interface (UI) 116, such as an integrated display device for displaying information to the user, and in some embodiments, receiving information from the user. For example, the user interface 116 may include a touchscreen and/or one or more input devices, such as buttons, knob or a keyboard that enable a user to provide an input.

In addition, the processor 114 may be operable to receive data or information from the analyte sensor 106 as well as other devices, such as smart accessory device 130, fitness device 133 or another wearable device 134 (e.g., a blood oxygen sensor or the like), that may be operable to communicate with the wearable drug delivery device 102. For example, fitness device 133 may include a heart rate sensor and be operable to provide heart rate information or the like.

The wearable drug delivery device 102 may interface with a network 108. The network 108 may include a local area network (LAN), a wide area network (WAN), a cellular network, or a combination thereof and operable to be coupled wirelessly to the wearable drug delivery device 102, the controller, and devices 130, 133, and 134. A computing device 132 may be interfaced with the network 108, and the computing device may communicate with the insulin delivery device 102. The computing device 132 may be a healthcare provider device, a guardian's computing device, or the like through which a user's controller 104 may interact to obtain information, store settings, and the like. The AID application 120 may be operable to execute an AID algorithm and present a graphical user interface on the computing device 132 enabling the input and presentation of information related to the AID algorithm. The computing device 132 may be usable by a healthcare provider, a guardian of the user of the wearable drug delivery device 102, or another user.

The drug delivery system 100 may include an analyte sensor 106 for detecting the levels of one or more analytes of a user, such as blood glucose levels, ketone levels, other analytes relevant to a diabetic treatment program, or the like. The analyte level values detected may be used as physiological condition data and be sent to the controller 104 and/or the wearable drug delivery device 102. The sensor 106 may be coupled to the user by, for example, adhesives or the like and may provide information or data on one or more medical conditions and/or physical attributes of the user. The sensor 106 may be a continuous glucose monitor (CGM), ketone sensor, or another type of device or sensor that provides blood glucose measurements that is operable to provide blood glucose concentration measurements. The sensor 106 may be physically separate from the wearable drug delivery device 102 or may be an integrated component thereof. The analyte sensor 106 may provide the processor 114 and/or processor 119 with physiological condition data indicative of measured or detected blood glucose levels of the user. The information or data provided by the sensor 106 may be used to modify an insulin delivery schedule and thereby cause the adjustment of drug delivery operations of the wearable drug delivery device 102.

In the depicted example, the controller 104 may include a processor 119 and a memory 128. The controller 104 may be a special purpose device, such as a dedicated personal diabetes manager (PDM) device. The controller 104 may be a programmed general-purpose device that is a portable electronic device, such as any portable electronic device, smartphone, smartwatch, fitness device, tablet or the like including, for example, a dedicated processor, such as processor, a micro-processor, or the like. The controller 104 may be used to program or adjust operation of the wearable drug delivery device 102 and/or the sensor 106. The processor 119 may execute processes to manage a user's blood glucose levels and control the delivery of the drug or a therapeutic agent (e.g., a liquid drug or the like as mentioned above) to the user. The processor 119 may also be operable to execute programming code stored in the memory 128. For example, the memory 128 may be operable to store an AID application 120 for execution by the processor 119. The AID application 120 may be responsible for controlling the wearable drug delivery device 102, including the automatic delivery of insulin based on recommendations and instructions from the AID algorithm, such as those recommendations and instructions described herein.

The memory 128 may store one or more applications, such as an AID application 120, a voice control application 121, and audio 139 which may be the same as, or substantially the same as those described above with reference to the insulin delivery device 102. In addition, the settings 121 may store information, such as drug delivery history, blood glucose measurement values over a period of time, total daily insulin values, and the like. The memory 128 may be further operable to store audio data and/or audio processing computer programs 139, such as audio messages, natural language processing code and libraries and the like. In addition, the memory may store AID settings and parameters, insulin treatment program history (such as insulin delivery history, blood glucose measurement value history and the like). Other parameters such as insulin-on-board (IOB) and insulin-to-carbohydrate ratio (ICR) may be retrieved from prior settings and insulin history stored in memory. For example, the AID application 120 may be operable to store the AID algorithm settings, such as blood glucose target set points, insulin delivery constraints, basal delivery rate, insulin delivery history, wearable drug delivery device status, and the like. The memory 128 may also be operable to store data such as a food database for carbohydrate (or macronutrient) information of food components (e.g., grilled cheese sandwich, coffee, hamburger, brand name cereals, or the like). The memory 128 may be accessible to the AID application 120 and the voice control application 121.

The input/output device(s) 143 of the controller 104 may include one or more of a microphone, a speaker, a vibration device, a display, a push button, a tactile input surface, or the like. The input/output device(s) 143 may be coupled to the processor 119 and may include circuitry operable to generate signals based on received inputs and provide the generated signals to the processor 119. In addition, the input/output device(s) 143 may be operable to receive signals from the processor 119 and, based on the received signals, generate outputs via one or more respective output devices, such as a speaker, a vibration device, or a display.

The controller 104 may include a user interface (UI) 123 for communicating visually with the user. The user interface 123 may include a display, such as a touchscreen, for displaying information provided by the AID application 120 or voice control application 121. The touchscreen may also be used to receive input when it is a touch screen. The user interface 123 may also include input elements, such as a keyboard, button, knob, or the like. In an operational example, the user interface 123 may include a touchscreen display controllable by the processor 119 and be operable to present the graphical user interface, and in response to a received input (audio or tactile), the touchscreen display is operable present a graphical user interface related to the received input.

The controller 104 may interface via a wireless communication link of the wireless communication links 198 with a network, such as a LAN or WAN or cellular network or combination of such networks that provides one or more servers or cloud-based services 110 via communication circuitry 122. The communication factor circuitry 122, which may include transceivers 127 and 125, may be coupled to the processor 119. The communication circuitry 122 may be operable to transmit communication signals (e.g., command and control signals) to and receive communication signals (e.g., via transceivers 127 or 125) from the wearable drug delivery device 102 and the analyte sensor 106. In an example, the communication circuitry 122 may include a first transceiver, such as 125, that may be a Bluetooth transceiver, which is operable to communicate with the communication circuitry 122 of the wearable drug delivery device 102, and a second transceiver, such as 127, that may be a cellular transceiver, a Bluetooth® transceiver, a near-field communication transceiver, or a Wi-Fi transceiver operable to communicate via the network 108 with computing device 132 or with cloud-based services 110. While two transceivers 125 and 127 are shown, it is envisioned that the controller 104 may be equipped more or fewer transceivers, such as a cellular transceiver, a Bluetooth transceiver, a near-field communication transceiver, or a Wi-Fi transceiver.

The cloud-based services 110 may be operable to store user history information, such as blood glucose measurement values over a set period of time (e.g., days, months, years), a drug delivery history that includes insulin delivery amounts (both basal and bolus dosages) and insulin delivery times, types of insulin delivered, indicated meal times, blood glucose measurement value trends or excursions or other user-related diabetes treatment information, specific factor settings including default settings, present settings and past settings, or the like.

Other devices, like smart accessory device 130 (e.g., a smartwatch or the like), fitness device 133 and other wearable device 134 may be part of the drug delivery system 100. These devices may communicate with the wearable drug delivery device 102 to receive information and/or issue commands to the wearable drug delivery device 102. These devices 130, 133 and 134 may execute computer programming instructions to perform some of the control functions otherwise performed by processor 114 or processor 119. These devices 130, 133 and 134 may include user interfaces, such as touchscreen displays for displaying information such as current blood glucose level, insulin on board, insulin deliver history, or other parameters or treatment-related information and/or receiving inputs. The display may, for example, be operable to present a graphical user interface for providing input, such as request a change in basal insulin dosage or delivery of a bolus of insulin. Devices 130, 133 and 134 may also have wireless communication connections with the sensor 106 to directly receive blood glucose level data as well as other data, such as user history data maintained by the controller 104 and/or the wearable drug delivery device 102. The embodiments presented herein may also be performed by a plurality of processors for example in a distributed computer system.

The user interface 123 may be a touchscreen display controlled by the processor 119, and the user interface 123 is operable to present a graphical user interface that offers an input of a subjective insulin need parameter usable by the AID application 120. The processor 119 may cause a graphical user interface to be presented on the user interface 123. Different examples of the graphical user interface may be shown with respect to other examples. The AID application 120 may generate instructions for the pump 118 to deliver basal insulin to the user or the like.

The processor 119 is also operable to collect physiological condition data related to the user from sensors, such as the analyte sensor 106 or heart rate data, for example, from the fitness device 133 or the smart accessory device 130. In an example, the processor 119 executing the AID algorithm may determine a dosage of insulin to be delivered based on the collected physiological condition of the user and a specific factor determined based on the subjective insulin need parameter. The processor 119 may output a control signal via one of the transceivers 125 or 127 to the wearable drug delivery device 102. The outputted signal may cause the processor 114 to deliver command signals to the pump 118 to deliver an amount of drug related to the determined dosage of insulin in the reservoir 111 to the user based on an output of the AID algorithm. The processor 119 may also be operable to perform calculations regarding settings of the AID algorithm as discussed as herein. Modifications to the AID algorithm settings provided via the voice control application 121, such as by the examples described herein, may be stored in the memory 128.

Control of the AID algorithm processes and AID application functions described above and below may advantageously be performed by using voice or audio commands that enable users to control delivery of therapeutic drugs hands-free. The voice control application 121 and AID application 120 of the controller 104 and the voice control application 188 and AID application 126 of the wearable drug delivery device 102 may operate in cooperation with one another to enable the voice control functions described with reference to the examples illustrated in FIGs. 2-16. In addition, or alternatively, the voice control application 121 and AID application 120 of the controller 104 may operate without interaction with the voice control application 188 or the AID application 126 to control the operation of the wearable drug delivery device 102. Similarly, the voice control application 188 and AID application 126 of the wearable drug delivery device 102 may operate without interaction with the voice control application 188 or the AID application 120.

In an operational example, the voice control application 121 when executed by the processor may be operable to manage receipt and processing of the audio input applying natural language processing (NLP) of the voice control application 121 on any inputs received by the input/output device 143 (e.g., a microphone).

The audio processing computer programs resident on the controller 104 and wearable drug delivery device 102, either in isolation or in combination, may individually or in cooperation with each other, control the AID application 120 on the processor 119 or AID application 126 on the wearable drug delivery device 102. In addition to storing user history information, the cloud-based services 110 may also be responsive to inputs provided via the voice control applications 121 and 188. It is envisioned that the configuration of the voice control applications 121 and 188 enable cooperative interaction with the respective AID applications 120 and 126. In addition, it is further envisioned that the voice control application 121 and the voice control application 188 may operate separately to enable control of the wearable drug delivery device 102 when the controller 104 is unavailable, such as when not conveniently located for use by the user or when not communicatively coupled to the wearable drug delivery device 102.

FIG. 2 illustrates an example of a wearable drug delivery device configured to provide audio messages according to the described examples.

For ease of illustration and discussion with respect to the example of FIG. 2, all of the elements of an AID system, such as those explained above with reference to the example system 100 of FIG. 1, are not shown. The voice control aspects of the AID system 200 as shown in FIG. 2 may include a portable electronic device 210 and a wearable drug delivery device 220. The portable electronic device 210 may be a controller, such as controller 104, that is implemented on a personal diabetes management (PDM) device, a smartphone, a tablet computer, or the like. A PDM may, for example, have a form factor of a smartphone but does not have all of the functions of a smartphone. The portable electronic device 210 may include a microphone/speaker 211, communication circuitry 212, a processor 213, an AID application 214, and a voice control application 215. In addition, in some examples, the portable electronic device 210 may further include a natural language understanding (NLU) module 217. The NLU module 217 may be operable to perform natural language processing (NLP) functions, such as recognize or interpret conversational audio inputs, including those that are composed of phrases and sentences that include slang, mispronunciations, and transposed words. The voice control application 215 may have limited NLP ability to recognize and interpret keywords, key phrases, codewords and phrases. Note that the natural language understanding module 217 may also be operable to provide natural language processing as well as natural language generation (e.g., text-to-speech and *vice versa*).

The wearable drug delivery device 220 may include sound chip circuitry 230 and an AID system 250. The AID system 250 may encompass all elements of the wearable drug delivery device 102 of FIG. 1 including those that are not shown in this example.

The sound chip circuitry 230 may include a microphone and speaker (microphone/speaker 232), a recording memory 234, a power source (standalone or from the AID system) 236, voice control circuitry/application 238 with inputs and outputs, and, in some examples, a natural language understanding (NLU) module 237. In addition, the sound chip circuitry 230 may include a vibration device 239 operable to generate a vibration or a sequence of vibrations that signal different information or operations (e.g., bolus delivery, command receipt confirmation or the like) that may be felt or heard by a user.

The microphone/speaker 232 may include circuitry operable to receive spoken words and convert the spoken words into electrical signals that may be stored by the voice control circuitry/application 238 in the recording memory 234 and/or processed by the voice control circuitry/application 238. In addition, the microphone/speaker 232 may be operable to output sounds via a speaker. The recording memory 234 is operable to store audio clips and audio messages that have either been pre-recorded, such as musical clips, stories, or the like. For example, the pre-recorded messages may include audio or messages that have not been recorded by a user, a user's guardian or relative, or the user's healthcare provider, but may be messages that have been recorded by a commercial enterprise (such as creators of children's movie character, sports figures or organizations, or the like) for distribution. In addition, or alternatively, the recording memory 234 may be able to store audio clips and audio messages recorded by the user, the user's guardian or relative or the user's healthcare provider.

The power source 236 may be a battery, supercapacitor, or other source of electrical power. The voice control circuitry/application 238 may be software programs, hardware, firmware, an application-specific integrated chip, a processor with access to software programs, a combination of the foregoing, or the like. The voice control circuitry/application 238 may be a processor operable to execute a software program that may be an instance of voice control application 215. In some examples, the voice control circuitry/application 238 is operable to provide the same functionality as the voice control application 215 when executed by a processor of the voice control circuitry/application 238. The voice control circuitry/application 238 may be operable to receive the electrical signals from the microphone/speaker 232, interpret the electrical signals and produce a response to the interpreted electrical signal, such as storing the electrical signal as part of a recorded message, provide control signals, such as causing the recording of the message, generating and forwarding a command to the AID system 250, or the like. In some embodiments, in addition to the previous components, the sound chip circuitry 230 may include a natural language understanding (NLU) module 237 that is operable to recognize or interpret conversational audio inputs, including those that are composed of phrases and sentences that include slang, mispronunciations and transposed words. Note that the natural language understanding module 237 may also be operable to provide natural language processing as well as natural language generation.

In order to enable the generating and forwarding of a command to the AID system 250, the microphone of the microphone/speaker 232 may be operable to detect key phrases spoken by a user and generate corresponding electrical signals. The corresponding electrical signals may be processed by the voice control circuitry 238, the natural language understanding module 237, and/or the processor 254 of the AID system 250 to identify the command to be generated and forwarded to the AID system 250. Examples of the command may be inquiries of "a latest bolus dosage," "an amount of insulin remaining in a reservoir of the wearable drug delivery device," "a current setting of the wearable drug delivery device," "a previous basal delivery rate," "a blood glucose measurement value," "a most recent blood glucose measurement value," "a trend of the user's blood glucose measurement values," or the like. Other exemplary commands, as explained in further detail below, include commands to perform an action or a change of a setting of the AID system, such as change a basal rate or deliver a meal or correction bolus.

Audio commands may also be provided wirelessly via a microphone 211 or 232. Alternatively, or in addition, the sound chip circuitry 230 may enable storage of pre-recorded audio clips (or may be downloaded at time of manufacture of the sound chip circuitry 230 or at initial use of the wearable drug delivery device 220). The recording memory 234 may be a random access memory or the like operable to store a number of recorded messages including snippets of musical scores, songs, phrases, and the like.

Operationally, the sound chip circuitry 230 may, for example, have preloaded audio messages stored in the recording memory 234 as well as being operable to record custom audio messages directly on the wearable drug delivery device 220 via the voice control circuitry 238, or a voice control application 215 on a portable device 210, such as a personal diabetes manager device, a controller, a smartphone, or the like. The recording memory 234 is operable to store both pre-recorded messages as well as custom recordings in respective files under control of the voice control circuitry 238 and/or voice control application 215. For example, the prerecorded messages (e.g., recorded by a commercial enterprise for distribution) and/or custom recordings may be stored in the recording memory 234 of the sound chip circuitry 230 on the wearable drug delivery device 220 by using the communication circuitry 252 coupled to the AID system 250 of the wearable drug delivery device 220. The voice control circuitry 238 may be operable to receive voice inputs of the message to be recorded via the microphone/speaker 232 and store a recording of the message in the recording memory 234. In a further example, the voice control circuitry 238 may interact with the processor 254 of the AID system 250 to enable the recording of the message by distributing processing between the voice control circuitry 238 and the processor 254.

In a further example, the portable electronic device 210 may be operable to execute the voice control application 215 that enables the processor 213 via the communication circuitry 212 to interact with the communication circuitry 252 of the wearable drug delivery device 220 to receive a previously recorded message from an external source, such as cloud-based services 110 of FIG. 1.

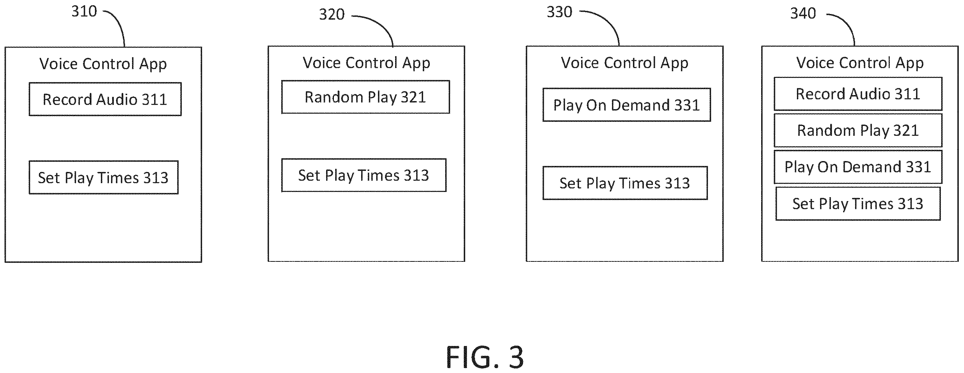

In addition to voice commands as discussed above, the voice control application 215 may be activated by touch commands. In a touch command operational example described with reference to FIGs. 2 and 3, the accelerometer 289 of the wearable drug delivery device 220 may be operable to enable the implementation of a tactile user interface. The tactile user interface implemented via the accelerometer 289 enables a touch or a press interaction with either a specific surface (or surfaces) on the wearable drug delivery device, or with any surface of the wearable drug delivery device that is operable to cue the playing of the stored audio clips or to wake-up or prepare the device to receive an audio command from the user.

For example, with reference to FIG. 2, a portion of a top surface of the wearable drug delivery device 220 may be configured as the tactile user interface and may incorporate or be electrically coupled with the accelerometer 289. The voice control application 238 may be tuned to generate specific outputs in response to the tactile user interface being tapped. In an example, in response to a specific touch or touch sequence (e.g., tap-tap-press, tap-tap-tap, various tap-pause combinations, or the like), the sound chip circuitry 230 may playback a recorded message. In addition, or alternatively, the specific touch or touch sequence may be a selection of a setting, such as set play times for the audio clips to be played, a selection of random play functions, or a selection of an on-demand function. When the setting is to play the recorded audio clips at either set play times or at random play, the recorded audio clips may cycle through the available selections either according to the respective set play times or according to a random play algorithm. In a further example, the voice control application 238 may generate an audio confirmation request, which is a request to confirm a latest voice input command or request. The tactile user interface may be used to provide the requested confirmation in response to an audio confirmation request.

FIG. 3 illustrates a block diagram representation of recording and playback functions provided by exemplary voice control circuitry and applications such as those shown in the example wearable drug delivery device of FIG. 2. The voice control circuitry/application 238 executing on a wearable drug delivery device may be configured with a number of recording and playback settings and functions. The different settings and functions may be selectively enabled via a user interface on the portable electronic device 210, which provides the selected settings and functions to the voice control circuitry/application 238 of wearable drug delivery device 220 for output. For example, via interaction with the voice control application 215, different settings and functions may be enabled on the wearable drug delivery device 220. For example, different versions of a collection of settings (e.g., to implement a particular mode of operation) may be downloaded to the wearable drug delivery device 220. The particular mode of operation may, for example, be a sleep mode (with nighttime settings), an activity mode (with exercise-related settings), a hypo-protect mode (with reduced drug delivery and/or higher target BG settings), a meal mode (with mealtime related settings), or the like.

In the example of FIG. 3, a version of the voice control circuitry or voice control application, voice control application 310 may present options to record audio 311 and set the play times 313 of the recorded audio. The presentation may be visually, if coupled to a portable electronic device equipped with a display, audibly, if coupled to a portable electronic device equipped with a speaker, or both, if coupled to a portable electronic device equipped with both a display and a speaker. Alternatively, voice control application 320 may present options to randomly play 321 stored audio and set the play times 313 of the stored audio. In a further alternative, voice control application 330 may present options to play the stored audio on demand 331 or set the play times 313 of the stored audio. In a final alternative, a full complement of functions may be provided by an example of the voice control application 340, which may provide the functions of record audio 311, randomly play 321, play on demand 331 and set the play times 313. The selected playback options may be relayed to the sound chip circuitry of the wearable drug delivery device for implementation and playback of the respective audio clips or messages.

For example, the recording and playback settings and functions may include a setting that enables recording of a number of audio clips (i.e., audio messages or music, such as workout music, a workout routine, a podcast, or the like). Alternatively, or in addition, the sound chip circuitry may come with pre-recorded audio clips. Whether the audio clips are recorded by a user or pre-recorded (e.g., recorded by a commercial enterprise for distribution) and downloaded from an external source (such as a computer, a cloud-based service, website, or the like) and setting playtime for the recorded audio clips, a random play setting that may provide a "shuffle" feature that plays the recorded audio clips in a random order (as opposed to the order in which the audio clips were recorded, were stored, or ordered for play by a user, user's guardian, or health care provider), or a play-on-demand setting (i.e., a setting that allows users to select specific recorded audio clips to be played out of the number of recorded audio clips.

By way of example, the voice control application on the portable electronic device may be used to record a set of lullabies, rhymes, short stories/ simple messages that may be provided to the wearable drug delivery device for playing at another time (such as bedtime, bath time, nap time), or at a random time (e.g., telling a story while traveling). A lullaby may be primarily for a pediatric user, and may be played at a set play time, e.g., 313, such as bedtime or nap time. The specific choice of lullaby may be selected in a voice control application preferences menu (not shown). The rhymes may be morning or bedtime rhymes that may help a pediatric user or elderly user get ready in the morning or at bedtime by presenting the list of tasks that need to be done to get ready for their day or for bed. The short stories may, for pediatric patients, provide comfort or occupy the user for a short period of time. Simple messages may include "Good Morning," "Have a great day today," 'Good Evening," "Good Afternoon," "Good night," and the like. It is also envisioned that the recorded audio clips may fall into a number of categories, such as lullabies, nursery rhymes, statements of praise (e.g., "good job, you stayed in range today," "you are in control of your insulin today," or the like), or short stories (which may be age appropriate for the user).

It is also envisioned that the voice control application of the sound chip circuitry (as shown in FIGs. 1-16) are operable to provide informational messages that may be helpful for some users, such as the elderly. In particular, patients with diabetes may suffer from diabetic retinopathy and have pronounced visual impairments. Diabetic retinopathy may occur in Type 1 and Type 2 diabetic patients. Blood vessels in the eyes may be damaged due to diabetic retinopathy causing visual impairment. The embodiments disclosed herein may assist users who are visually impaired. For example, the voice control application may provide notifications and/or alerts, such as bolus announcements (e.g., "It is time to administer a bolus."), blood sugar measurement value announcements, other measurement or calculated value announcements, time of day messages, and/or status notifications. For example, the bolus announcements/blood sugar announcements, may benefit elderly users by providing the audio feedback on a delivered bolus, blood sugar levels and trends, feedback on when to eat, measure their blood glucose, or the like. The messages may also be time of day messages offering encouragement or empathy that may provide some comfort to elderly patients through the day.

Some of the playback settings may be triggered by other actions or other contexts. For example, a context for triggering a set play time 313 for a child may be when the child's portable electronic device is placed in or on a docking station near the child's bed for recharging, or when a particular Wi-Fi or Bluetooth signal strength indicative of the child being in their bedroom is detected. For example, the output of signal strength circuitry of the portable electronic device may be accessible to the voice control application to enable location determination based on signal strength, such as Wi-Fi or Bluetooth.

Blind diabetic users are another set of patients where voice control and audio feedback play a vital part. Without audio interfaces, blind diabetics will need help from additional caregivers to manage their diabetes.

Yet another category of diabetic users who will benefit from audio interfaces include patients who may have had limb amputations, paralysis of the limbs, motion impairment, for example, due to tremors arising with Parkinson's disease, and the like.

In one playback example, each of the voice control applications 310-340 may be operable to selectively trigger a specific audio clip based on a time of day/random play, or the like. For example, the voice control applications 310-340 executed by a processor may be operable to maintain a clock and access voice control application user preference settings related to the respective function. These different features may be selectively set or provided based on different criteria, such as age of the user, number of audio recordings stored in memory, or the like.

In addition, or alternatively, a voice or alarm control application on the portable electronic device may also allow alarms to be set by the user using the portable electronic device. In some examples, the alarm may be a vibration of the user's wearable drug delivery device instead of an audio alarm, so as to avoid, for example, waking a spouse, disturbing others, or alerting others of the alarm, or the like. For example, the voice or alarm control application may, in response to voice commands, present a menu similar to the set play times 313 menu of FIG. 3, but instead of setting music/message play times, the user has the opportunity to set an alarm or reminder times for making a blood glucose measurement, to consume a meal, inputting a bolus dosage amount, present an indication when the user's blood glucose goes below a certain user-set threshold, or the like. Alternatively, the alarm may be a time alarm that the user may set, and at the alarm time the wearable drug delivery device may vibrate so as to alert the wearer without alarming other people nearby with an audio alarm, such as early in the morning or in a meeting or movie theater.

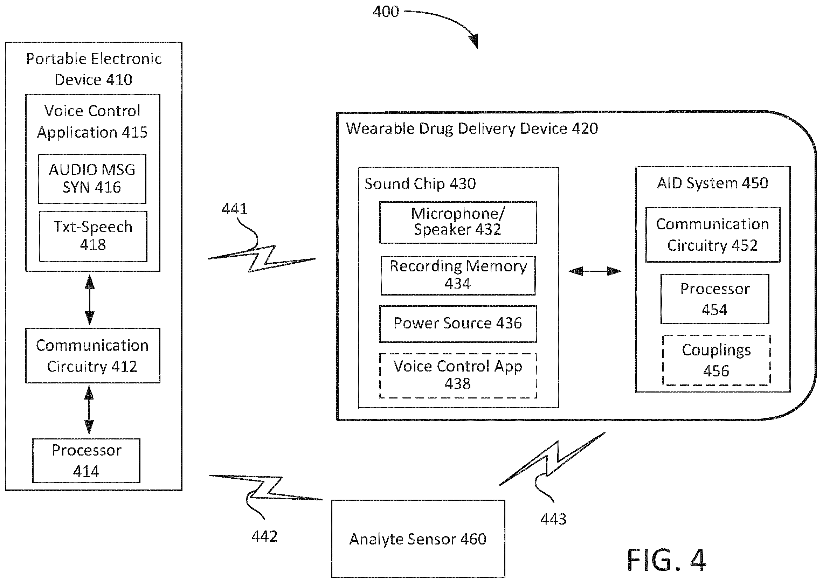

FIG. 4 illustrates another block diagram representation of an example of AID system configured to provide audio messages according to the described examples.

In the example of FIG. 4, the AID system 400 may include a portable electronic device 410, such as a PDM or an appropriately equipped smartphone, an analyte sensor 460, such as a continuous glucose monitor (CGM) or ketone monitor, and a wearable drug delivery device 420.

The portable electronic device 410 may include communication circuitry 412, a processor 414, a memory (not shown), and a voice control application 415 that may perform functions similar to those described with reference to the examples of FIGs. 1 and 2.

Additionally, the voice control application 415 may utilize a voice verification and security protocol(s), which may be integrated into the voice control application. For example, the voice control application 415 may upon an initial use, or after a period of time, implement a voice enrollment process. For example, the user's voice may be enrolled via the portable electronic device 410. The user may be asked to speak chosen representative sentences, for example - "Today is Monday and the weather outside is beautiful." The spoken sentences are used to create a voice signature for the respective user according to a voice verification and security protocol(s) implemented by the voice control application 415. The respective voice signature(s) may be stored on the portable electronic device 410, and also communicated to the wearable drug delivery device 420 or a new wearable drug delivery device at the time when the wearable drug delivery device 420 is changed.

In addition, the voice control application 415 may also be operable to perform a speaker (of verbal commands) verification and authentication process according to know verification and authentication protocols. For example, when a user tries to give audio commands to the wearable drug delivery device 420 or the portable electronic device 410, a first step may be voice authentication. The speaker's voice may be validated against the stored voice signature and authenticated. If the speaker is authenticated, audio commands may be issued by the authenticated speaker, and followed by the voice control application 415 executing on the portable electronic device 410 or the voice control application 438 executing on the wearable drug delivery device 420. As a further security feature, each command may also be voice authenticated before being accepted, and followed.

As a security feature, the voice enrollment/verification/authentication sequences implemented at the start of accepting audio commands enables a high level of security for the AID system 400.

Another aspect is that the voice control application 415 may adapt over time. For example, as the user starts using audio commands, the voice signatures may be further enriched and updated using the new input. Changes in voice may thus be incorporated efficiently.

In a further example, the voice control application 438 of the wearable drug delivery device 420 is configured to implement the above described verification and authentication process as well as use the voice verification and security protocol(s).

For example, the communication circuitry 412 may include a number of transceivers that enable the portable electronic device 410 to communicate with the analyte sensor 460 via communication link 442 and with the wearable drug delivery device 420 via communication link 441. The analyte sensor 460 may be operable to detect physiological data of a user, such as blood glucose levels, ketone levels and the like, and be operable to transmit the detected physiological data to the wearable drug delivery device 420. The processor 414 may be coupled to the communication circuitry 412 to receive signals from and transmit signals to the analyte sensor 460 and the wearable drug delivery device 420. The portable electronic device 410 may include all of the features of and be operable to perform the functions of the controller 104 of FIG. 1; however, for ease of discussion, not all of the features shown in controller 104 of FIG. 1 are shown or described with reference to the portable electronic device 410.

The wearable drug delivery device 420 may be similar to the wearable drug delivery device 102 of FIG. 1, but not all of the features shown in wearable drug delivery device 102 of FIG. 1 are shown or described with reference to wearable drug delivery device 420. For example, the wearable drug delivery device 420 includes a sound chip 430 and an AID system 450. The AID system 450, in this example, is shown to include communication circuitry 452 and a processor 454. In some examples, the AID system 450 may include optional couplings 456 that, in some examples, couples to packaging that contains the wearable drug delivery device 420. In addition, the voice control application 415 may include additional modules (where a module may be programming code, circuitry, or a combination thereof) directed to audio message synthesis 416 and a text-to-speech module 418. In some examples, the sound chip 430 may utilize the functionality of communication circuitry 452 and the processor 454 to accomplish one or more tasks or functions. The communication circuitry 452 may include one or more wireless communication transceivers, such as a wi-fi transceiver, a cellular transceiver, a Bluetooth transceiver, a near-field communication transceiver, or the like.

Audio feedback from the voice control application 415 or the sound chip 430 can be provided in the form of audio messages such as "Bolus Delivered", blood glucose level announcements, assurance messages, such as "Blood glucose is in good range," "Blood sugar going low," "Eat some rescue carbohydrates," and the like. The messages also may be status messages, such as, for example, the amount of insulin remaining in the reservoir, the remaining duration of wearable drug delivery device power supply, a last bolus amount, an amount of insulin delivered so far in the day, the amount of insulin delivered in the past (e.g., yesterday or one week ago), current insulin on board (IOB), the presence of an occlusion or of a potential occlusion, a communication issue with the CGM, a communication issue with a portable electronic device, an out of communication range for the wearable drug delivery device with respect to a portable electronic device and/or a CGM, or the like. The audio feedback provided by the voice control application 415 and sound chip 430 improves the capabilities of the AID system 400 to communicate relevant information with the user, especially if they are elderly, by allowing the user to obtain status information without having to remember to request such information or fumbling around with the portable electronic device to obtain the status information. As mentioned earlier, diabetic retinopathy in the elderly may cause significant visual impairment. The voice control application 415 may assist with these and other users by being further operable to monitor blood glucose measurements and provide appropriate audio feedback to the user via the sound chip 430.

In a further example, a wearable drug delivery device 420 optionally equipped with the voice control application 438 may be operable to assist a user with unpackaging, assembling (if necessary), and initializing the wearable drug delivery device for use by the user. In this example, the wearable drug delivery device 420 may initially be provided to the user in packaging (not shown). The wearable drug delivery device 420 may, for example, have optional couplings 456 to the packaging. Examples of couplings 456 could be photosensors, non-conductive transparent strips that are pulled from between circuit contacts or battery contacts, pins that apply pressure to a button, or the like. When the couplings 456 are broken or triggered, the wearable drug delivery device 420 may be energized (i.e., power is supplied from a power source to the wearable drug delivery device) and the voice control application may be launched.

In an operational example, upon launch of the voice control application 438, the sound chip circuitry 430 may receive an unpackaging indication (such as supply of power from the power source 436) that the package containing the wearable drug delivery device has been opened and one or more of the couplings 456 have been triggered. The sound chip circuitry 430 may use the unpackaging indication to identify a specific audio clip from the plurality of audio clips stored in the recording memory 434. For example, the identified specific audio clip may include instructions related to initializing the wearable drug delivery device 420 for use by the user. More specifically, the voice control application 438 may be operable to communicate via the wearable drug delivery device's speaker 432 with the user, for example, the voice control application 438 may cause the output of audio messages that welcome the user and guide the user through a setup or initialization process for the wearable drug delivery device 420.

In a further example, the audio clip (also referred to interchangeably as "audio messages" throughout the application) may also provide a training audio or more detailed guidance for the setup process. In addition, the voice control application 415 of the portable electronic device 410 may be operable to detect the state of the wearable drug delivery device 420 and inform the user of next steps in the set-up process (which may include positioning and placing the wearable drug delivery device on the skin of the user and configuring the wearable drug delivery device for delivery of insulin).

In yet a further example, the voice control application 415 and the sound chip 430 may be operable to transfer messages between one another. For example, a parent may use the portable electronic device to communicate via the voice control application 415 with their child who is a user of the wearable drug delivery device 420 with the sound chip 430. Messages from the parent may be transmitted to the child's wearable drug delivery device 420 and output by the sound chip 430. For example, the communication circuitry 412 of the wearable drug delivery device may be operable to relay messages (e.g., via a cellular, Bluetooth, or wi-fi transceiver) from the voice control application 415 to the communication circuitry 412, which transmits the message to the communication circuitry 452 of the wearable drug delivery device 420. The communication circuitry 452 via the processor 454 is operable to provide the relayed message to the sound chip 430 for output via the speaker 432. This feature is a particularly useful advantage over wearable drug delivery devices that are not so equipped because the wearable drug delivery device 420 is adhered to the child user and is always present so the child is substantially certain to receive the parent's message. The above advantages may also apply to when a child or an elderly person is lost (such occurrence can be common for elderly who have diabetes and Alzheimer's), confused, or the like.

Additional capabilities are also envisioned for a voice-controlled AID system and, in particular, a voice-controlled wearable drug delivery device. The voice control capability of an AID system and, in particular, a voice-controlled wearable drug delivery device to utilize voice commands, provides the advantage of allowing visually impaired users and those with limited manual dexterity (manual dexterity may be diminished with age and also impaired with illnesses such as Parkinson's) to utilize voice commands to control a number of AID system features. For example, the voice control capability may also facilitate functions such as, adjusting parameters of their AID system, bolusing, temporary basal rates, setting different modes of operation, such as setting an activity mode, locating misplaced controllers, direct verbal communication between the wearable drug delivery device without having to have the controller nearby, an ability to use the controller and wearable drug delivery device as verbal communication devices, and the like. It is further envisioned that free-form queries may be implemented by utilizing natural language understanding (NLU) of the voice commands. The voice commands may also allow for navigating a visual menu display as well as enabling natural language processing for personalization and reporting. For example, it is envisioned that the wearable drug delivery device 420, either with or without utilizing a controller 410, may be operable to provide audio-based voice command confirmation, maintain audio notes and annotations, and the like. Additionally, or alternatively, in this and in each of the other embodiments, the voice control application may cause the wearable drug delivery device to vibrate to confirm receipt of the voice command. For example, if a command or confirmation is successfully received, the wearable medical device may vibrate three short times; and if a voice command or confirmation is not successfully received, the wearable medical device may vibrate once.

As part of the audio-based voice command confirmation, the wearable drug delivery device 420 or the controller (such as 410) may, upon activation of the voice commands functionality, receive a voice command, interpret a voice command, and generate control signals to implement an action to be taken, using voice control application 415 or 438. Before executing the action to be taken, the voice control application may request the user to provide audio feedback and confirmation of the action to be taken prior to executing the action to be taken. In addition, or alternatively, a user may record notes and annotations, for example, the user may make an audio description of a meal or a snack, a description of an exercise or activity, descriptions of feelings, and the like.

When utilizing a controller (such as 410), the user may be able to utilize the voice control application to navigate a menu on the controller (such as 410). The voice control application provides the capability for the user to navigate a voice driven menu. Similarly, the processing capability of a controller allows for natural language processing of the voice commands. This enables the user to personalize their controller and wearable drug delivery device as well as setting the reporting information and other user preferences. Notes and annotations may be automatically transcribed and processed with NLP techniques to generate insights and reports that may be emailed to a healthcare provider or to a user for evaluation or for archiving. The reporting information may include data about the controller (e.g., battery power level, and amount of user interaction with the voice control application and the AID application), the wearable drug delivery device (e.g., amount of drug left in the reservoir, battery power level, amount of drug delivered in a time frame (such as last hour, last day), amount of drug delivered in last bolus, and/or physiological information of the user (e.g., blood glucose measurement, ketone levels, insulin onboard, total daily insulin, and the like).

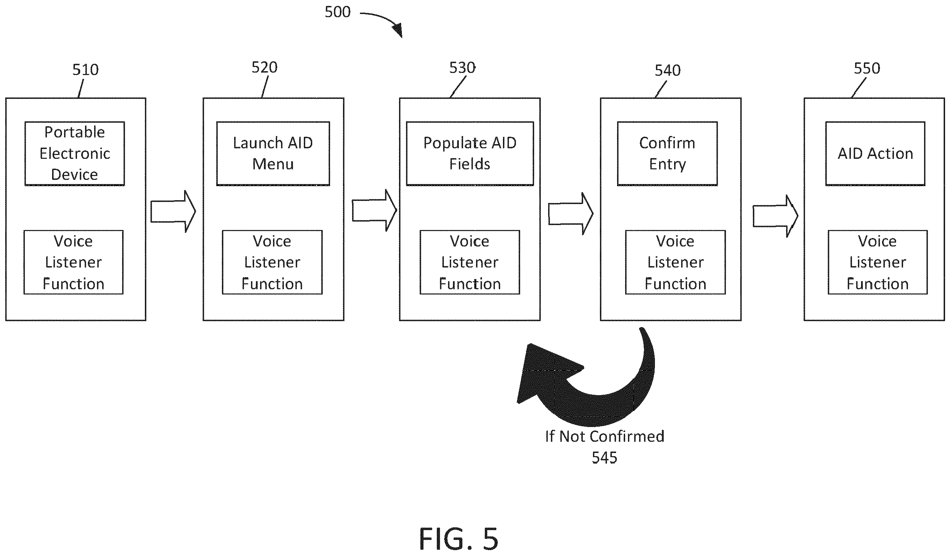

FIG. 5 illustrates an example of a voice control pipeline sequence for controlling an AID system according to the disclosed subject matter.

In the voice control pipeline sequence 500, AID voice commands may be used to control insulin delivery, including modes, volumes, times, durations, settings, notifications, measurements, and the like for the AID system. The voice control application may go through a series of actions based on audio inputs from the user as shown in FIG. 5. The AID systems shown in FIGs. 1, 2 and 4 configured with a portable electronic device, such as a controller, a smartphone or PDM, that has the voice control application installed, or with a wearable drug delivery device with a voice control application installed may be responsive to certain voice cues. For example, the portable electronic device or a wearable drug delivery device may be monitoring via a voice listening function of the installed voice control application, in a manner similar to Siri® and Alexa® for a keyword, key phrase, or the like. At 510, the portable electronic device may be in an active listening mode, waiting for the detection of a particular keyword, such as "Omnipod", a custom keyword, or the like.

In response to detection of the keyword, at 520, the voice control application is further activated, or fully activated, and causes the voice control application to launch an AID menu on an input/output device of the portable electronic device, such as a display device, a touchscreen display, or the like. In an example, the menu may include a selectable setting to increase a user's blood glucose target set point. At 530, the user may continue to provide voice commands to the voice control application. The voice control application may be operable to respond to keywords or key phrases (e.g., "set point") or be operable to interpret a command in the form of a conversational request in which a natural language unit is operable to process the command and ask further clarifying questions. The voice control application may be operable to respond to keywords or key phrases and numbers associated therewith, e.g. "change set point to 120" (mg/dL), Based on the interpretation of the command, the voice control application is operable to populate the menu. At 540, the voice control application may confirm the command. Confirmation of the command may require the user to provide a phrase or passcode, either orally or on the wearable drug delivery device 102, for example, by tapping a particular sequence on the housing. Alternatively, confirmation may entail the user confirming the command on the controller 104, e.g., by pressing a large "Confirm" button on UI 123 under details of the command issued by the user and populated on UI 123 of the controller 104 after voice control app 121 / 188, and its corresponding natural language processing, has interpreted the command. If the command is not confirmed, the voice control application proceeds to 545 and performs a repeat of 530. Alternatively, in response to the command being confirmed the voice control pipeline proceeds to 550. At 550, the command is executed and the appropriate insulin delivery or setting adjustment is provided and the voice control application may return to the active listening mode.

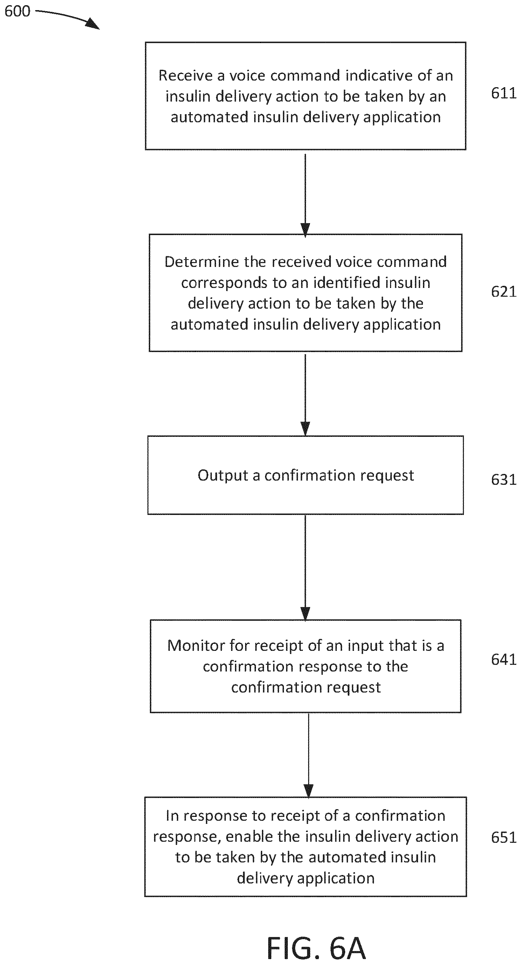

FIG. 6A is a flowchart of an exemplary general voice control process according to the disclosed subject matter. The general voice control process 600 may be utilized in a number of different voice control scenarios. The general voice control process 600 may be implemented by a portable electronic device, a wearable drug delivery device, a combination of the portable electronic device and the wearable drug delivery device. In addition, it is envisioned in some examples that external servers and/or cloud platform services may also be used in combination with the portable electronic device and the wearable drug delivery device.

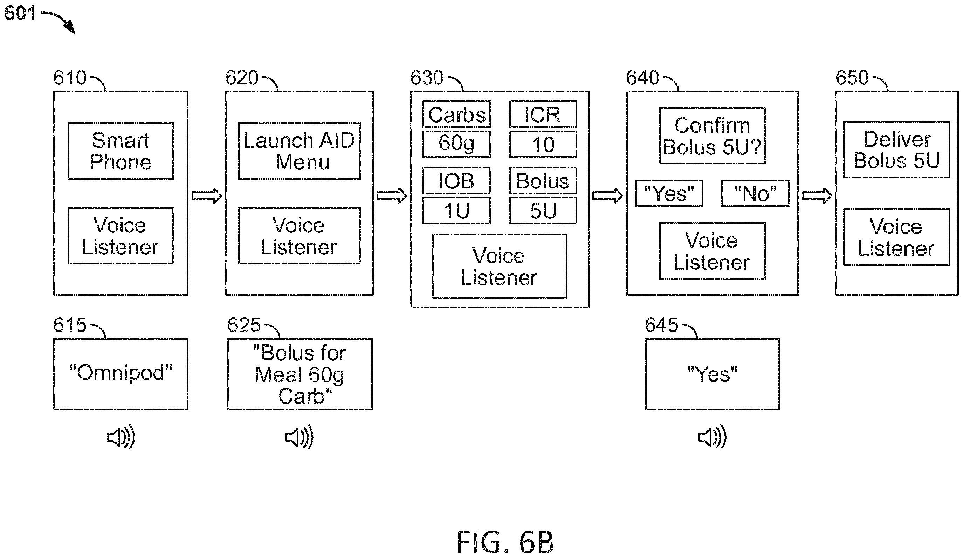

In an example of a wearable drug delivery device, such as those described with reference to earlier examples such as those of FIGs. 1 and 2, the wearable drug delivery device may include a processor, communication circuitry, sound chip circuitry and a memory. The sound chip circuitry may be operable to perform a number of functions. For example, at 611, the sound chip circuitry may be operable to receive via a microphone or via communication circuitry, a voice command indicative of an insulin delivery action to be taken by an automated insulin delivery application. Examples of voice commands are described in more detail with reference to examples that follow. At 621, the sound chip circuitry may determine the received voice command corresponds to an identified insulin delivery action to be taken by the automated insulin delivery application. For example, the sound chip circuitry may utilize natural language processing (described in the examples below) to recognize keywords or conversational phrases. The natural language processing may provide a recognition result that a voice control application of the sound chip circuitry may use to determine a corresponding insulin delivery action. The corresponding insulin delivery action may be presented visually to a display of a portable electronic device, audibly via a speaker of either the portable electronic device or the wearable drug delivery device, or both for consideration by the user. A confirmation request may be output (631) visually to a display of a portable electronic device, audibly via a speaker of either the portable electronic device or the wearable drug delivery device, both audibly and visually, via a vibration sequence, or a combination of the three. Additionally, or alternatively, the voice control application may cause the wearable drug delivery device to vibrate to request confirmation, where the vibration replaces or supplements the audio confirmation request message thereby further enhancing the systems usability with those with dexterity or vision impairments. At 641, the sound chip circuitry may be operable to monitor for receipt of the confirmation response, which may be a keyword audio input, which is a response to the confirmation request. Examples of a confirmation response using keyword audio inputs is described in more detail with reference to the examples of FIGs. 6B-16. In response to receipt of a keyword audio input as the confirmation response confirming the insulin delivery action, the sound chip circuitry may enable the insulin delivery action to be taken by the automated insulin delivery application (651). The sound chip circuitry may enable the insulin delivery action, in one example, by forwarding a signal representing the corresponding insulin delivery action to an AID application for execution. The voice control application may return to an active listening mode.

FIG. 6B illustrates an example voice control progression for causing an AID system to deliver a bolus for a meal with known nutritional information according to the disclosed subject matter.

The sequence 601 of FIG. 6B shows an example of a voice command exchange with a voice control application hosted on a portable electronic device, such as a controller, a smartphone, or a PDM. For example, the portable electronic device may be monitoring for a keyword via an initial mode, such as an active listening mode, of the voice control application, in a manner similar to Siri® or Alexa®. In an example, the voice control application may be operable to utilize the natural language processing of either Siri or Alexa (generally referred to herein as a natural language understanding (NLU) module or application) provided on the portable electronic device to interpret the voice command exchange via a native voice command API.

At 610, the portable electronic device may be in an operational mode, called active listening mode, in which a voice listening function of the voice controller application is listening for a reduced subset of audio keywords, such as an activation keyword or an emergency keyword. Examples of the activation keyword may be audio inputs, such as "Omnipod," a custom keyword or key phrase (e.g., a particular name like "Frankie" or "Hey Wearable drug delivery device"), a codeword, such as "C9" or "AB", or the like. At 615, the activation keyword "Omnipod" or the like is detected by the voice listener function of voice control application. Note that the audio command may be provided to the portable electronic device wirelessly via a microphone integrated into Bluetooth-equipped personal listening/communication devices, via the microphone integrated in the portable electronic device (or wearable drug delivery device), or both.

In response to the detection of the activation keyword, at 620, the voice control application may switch to a full activation mode and cause the voice control application to launch an AID menu on an input/output device of the portable electronic device, such as a display device, a touchscreen display, or the like. In the full activation mode, the voice control application may expand the number of phrases, keywords, codewords (e.g., a full set of audio keywords) that the voice control application recognizes and interprets beyond the subset of audio keywords that are activation keywords.

The voice listener function remains active at 620 and detects the voice command phrase of "Bolus for Meal for 60 grams of Carbohydrate" (625) or the like. The voice command "Bolus for Meal for 60 grams of Carbohydrate" (625) refers to administering a bolus dosage to compensate for a meal the user consumed or intends to consume. As an alternative to detecting the voice command at 625, the voice command may be received at 625 by a remote microphone, such as a microphone housed in a hearing aid, ear buds, a headphone, or the like and transmitted to the portable electronic device and processed according to the operating system of the portable electronic device and the voice control application, an NLU application or applet, the AID application, and/or the like. The voice command 625 is interpreted according to known methods, such as a library of electronic signatures, an NLU application or applet or the like. For example, the NLU application or applet may parse the inputted voice command phrase for interpretation and recognition and, in response to the interpreted voice command, the voice control application may invoke a Bolus Menu. For example, the voice control application may use the output of the NLU application or applet to populate the Bolus menu with "60 g" for the carbohydrate field.

The Bolus Menu may be a routine that is called and causes a menu to be presented in a graphical user interface and is operable to receive inputs (tactile and/or verbal) to populate fields of the menu that describe the nutritional content of a meal, such as the number of grams of carbohydrates, amount of protein and/or fat. In addition, based on the output of the NLU application or applet, other values for parameters such as insulin-on-board (IOB) (e.g., 1U), and insulin-to-carbohydrate ratio (ICR) (e.g., 10) may be retrieved by the voice control application from prior settings and insulin history stored in memory (or from the AID application, or both) or may be calculated by the AID application, and populated in the Bolus Menu.

Returning to the operational example in FIG. 6B, the voice control application may be operable to pass a signal comprising the interpretation of "60 grams of carbohydrates" to the AID application. In response to receiving the number of grams of carbohydrates, the AID application may be operable to calculate a bolus dosage to compensate for the number of grams of carbohydrates and forward the calculated bolus dosage to the voice control application, which may populate the Bolus Menu. Or the AID application may populate the Bolus Menu directly with the calculated bolus dosage. For example, based on the example ICR and IOB values mentioned above, a bolus dosage of 5U may be calculated which accounts for 1U of insulin as IOB (60g Carb / 10 g/U - 1U) = (6U - 1U) = 5U. As shown in 630, the voice control application may cause a display of the controller to present the information obtained from the user as well as information calculated based on the information obtained from the user when calculating a meal bolus dosage, such as the number of carbohydrates in the meal (e.g., 60 grams), ICR (e.g., 10), IOB (e.g., 1 U), and the calculated bolus dosage (e.g., Bolus 5U), and the like. In addition, or alternatively, one or more of the values of ICR, IOB, number of grams of carbohydrates, and the calculated bolus dosage may be announced via a speaker. For example, the system could announce, "Ok, I have received your meal announcement for 60 grams of carbohydrates. I have calculated a bolus of 5 Units based on 60 grams of carbohydrates. Please confirm whether I should deliver 5 Units of insulin." In another example, the voice control application may output only the calculated bolus dosage to the speaker. Accordingly, the AID system may be configured to receive a voice command phrase including a number of carbohydrates, a request for a bolus, and/or calculate a bolus amount, and the AID system performs an action based on the received voice command phrase.

At 640, the voice control application may present the information on the display device of the controller as a confirmation graphical user interface, and, in addition or alternatively, cause a speech synthesizer (not shown in this example) to output a confirmation request message. An audio confirmation request message (e.g., "Confirm Bolus 5U") for delivery of 5U of insulin is presented visually and/or audibly. For example, the audio confirmation request message may be output by the voice control application as a visual message for presentation a confirmation graphical user interface in the display device of a controller, as an audio output from the speaker on a controller or a wearable drug delivery device, or as both the visual message and the audio output. In a hands-free audio control example, the audio confirmation request message indicates the action to be taken by the automated insulin delivery application and sets the voice control application for receipt of a confirmation keyword audio input related to the action to be taken by the automated insulin delivery application. The user may verbally state either "Yes" or "No" as a confirmation keyword, by way of example.

To provide additional drug delivery safety in some situations, the voice control application may be configured to require physical inputs (e.g., a tactile input to a graphical user interface or a surface of the drug delivery device as described earlier, and/or confirmation on UI 123 of controller 104 or UI 116 of drug delivery device 102) for confirmation of the proposed bolus dosage. If the additional drug delivery safety is in place, the user may respond to the confirmation request message by selecting either the "Yes" button or the "No" button, by way of example.

After confirmation, the proposed bolus dosage by receiving the input of "YES" at 645, a bolus of 5 U (in this example) may be delivered by the AID application. For example, the voice control application may deliver a signal to the AID application indicating that a 5 U bolus is to be delivered. At 650, the voice control application may cause the presentation that a bolus of 5 U has been delivered. The presentation may be by an audio output, a visual output, or by both. The voice control application may return to an active listening mode.

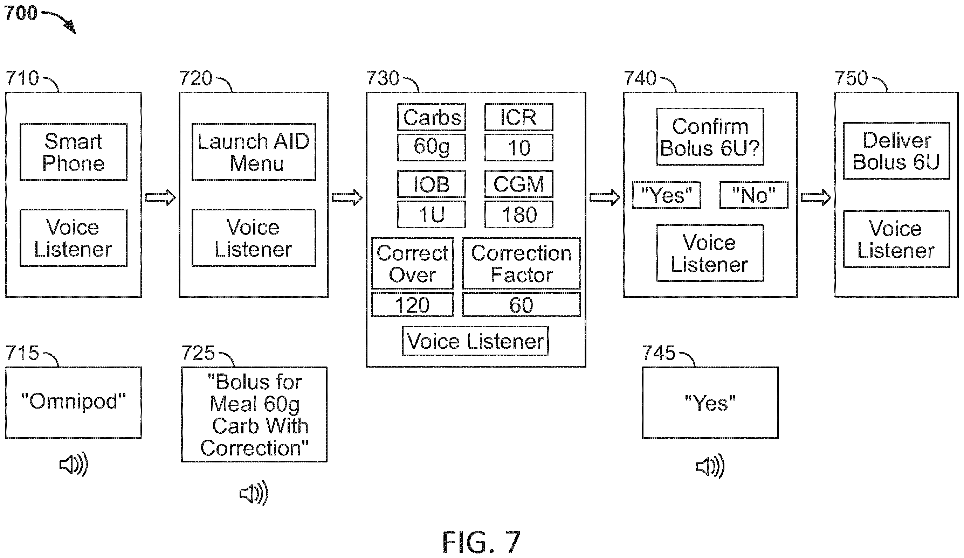

FIG. 7 illustrates an example voice control progression for causing an AID system to deliver a meal bolus with a correction dosage according to the disclosed subject matter.

Similar to the voice control action sequence 601 of FIG. 6B, the action sequence 700 of FIG. 7 begins at 710 with portable electronic device with an installed voice control application. As an initial operational mode, the voice control application may be in an active listening mode. In response to detecting the keyword "Omnipod" (715) at 710, the voice control application may be fully activated and begin listening for more complex commands other than keywords. In response to the detected keyword at 715, the voice control application may launch an AID menu, and the voice listener function remains active at 720 and detects the exemplary voice command phrase "Bolus for Meal for 60 grams of Carbohydrate with Correction" (725). The correction term, or "Correction," corresponds to a dosage of insulin that is delivered when the blood glucose measurement value is above an upper boundary (e.g., 1200 mg/dL), which may be the set point or target blood glucose value, or another chosen blood glucose value. As an example, in FIG. 7, the blood glucose value is 180 mg/dL which is 60 mg/dL over the upper boundary (shown as the "Correct Over" value in 730 and needs an insulin dose of (180 - 120)/Correction Factor or (180-120)/60 equivalent to 1 U of insulin. Note that the bolus (without correction) for the meal with 60 grams of carbohydrates is 5 U as described above with reference to FIG. 6B. The voice control application using the previously discussed voice recognition functions causes a routine, or routines, such as a Bolus Menu with a Correction Term routine, to be invoked. For example, the NLU application or applet of the voice control application may be operable to parse the phrase and populate the bolus menu with 60 g for Carbohydrate. Other parameters, such as ICR (Insulin to Carbohydrate Ratio), IOB (Insulin on Board), the blood glucose measurement value from the CGM and the like, may be retrieved from a prior setting, an insulin history, and/or a blood glucose history stored in memory. In the example, a bolus dose of 5U is calculated which accounts for 1U of insulin as IOB ((60g Carb / 10 g/U) - 1U) = (6U - 1U) = 5U. A correction dose of (180 - 120)/60 = 1U of insulin is added to the meal bolus dosage. The result is a bolus (5 U) with a correction dosage (1 U) for a total dosage of 6 U to be administered. Accordingly, the AID system may be configured to receive a voice command phrase for blood glucose value correction and calculate a bolus amount based on at least the current blood glucose value or current blood glucose value trend, and the target blood glucose value. It should be understood, that to calculate the bolus amount other factors such as correction factor, IOB, etc. are also used.

At 740, the voice control application may present the total dosage information on the display device of the controller, and, in addition or alternatively, cause a speech synthesizer (not shown in this example) to output a confirmation request message. A confirmation request message for delivery of 6U of insulin (e.g., "Confirm Bolus 6U") may be output to graphical user interface on the display device, output to a speaker as an audio output, or both. In response to the presentation of the confirmation request message, the user may respond by selecting either the "Yes" button or the "No" button via an input to the graphical user interface, or by responding verbally with a "Yes" or a "No." After confirmation by the input of "YES" at 745, a bolus of 6U is delivered. At 750, the voice control application may cause the presentation that a bolus of 6U has been delivered. The presentation may be by an audio output, a visual output, or by both. The voice control application may return to an active listening mode.

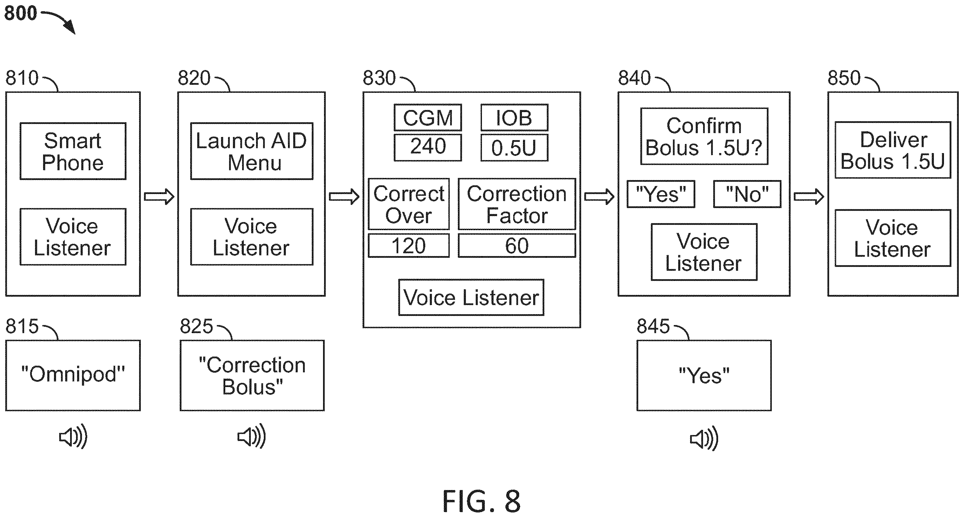

FIG. 8 illustrates an example voice control progression for causing an AID system to deliver a correction bolus according to the disclosed subject matter. This example is relevant to when a user has a high blood glucose measurement value and has not eaten a meal.

Similar to the voice control action sequences 601 of FIG. 6 and 700 of FIG. 7, the voice control action sequence 800 begins at 810 with portable electronic device with an installed voice control application. As an initial mode, the voice control application may be in an active listening mode. In response to detecting the keyword "Omnipod" (815) at 810, the voice control application may be fully activated and begin listening for more complex commands other than keywords. In response to the detected keyword at 815, the voice control application may launch an AID menu, and the voice listener function remains active at 820 and detects the voice command phrase "Correction Bolus," (825). A "Correction bolus" is not the same as a "meal bolus with correction" as described with reference to FIG. 7 above, instead a correction bolus corresponds to a dosage of insulin that is delivered when the blood glucose measurement value is above the upper boundary of the target blood glucose set point with the intent to bring the blood glucose measurement value below the upper boundary of the target blood glucose set point, or, more generally, to within range of the user's target blood glucose set point. The voice control application using the previously discussed voice recognition functions causes a routine, or routines, such as a Correction Bolus Menu routine, to be called.

For example, the command "Correction Bolus" (825) may cause a Correction Bolus Menu to be invoked. In this example, the NLU application or applet parses the phrase and populates the Correction Bolus menu with IOB (Insulin on Board), the blood glucose value from the CGM as well as the "correct over #" blood glucose settings and the correction factor. In some examples, the "correct over" is an AID application action in which the processor compares a current blood glucose measurement with a predetermined blood glucose value (i.e., a "correct over" value), which the processor uses to determine a number of units of insulin that the processor is correct. A correction dose of 1.5U is calculated which accounts for 2U of insulin for correction (240-120)/60 - 0.5U (IOB) = 1.5U.

At 840, the voice control application may present the 1.5U of insulin and other information on the display device of the controller, and, in addition or alternatively, cause a speech synthesizer (not shown in this example) to output a confirmation request message. For example, the confirmation request message (e.g., "Confirm Bolus 1.5 U") for delivery of 1.5 U of insulin may be output to graphical user interface on the display device, output to a speaker as an audio output, or both. In response to the presentation of the confirmation request message, the user may respond by selecting either the "Yes" button or the "No" button via an input to the graphical user interface, or by responding verbally with a "Yes" or a "No." After confirmation by the input of "YES" at 845, a bolus of 1.5 U is delivered. At 850, the voice control application may cause the presentation that a bolus of 1.5 U has been delivered. The presentation that the bolus is delivered may be by audio output, visual output, or by both. The voice control application may return to an active listening mode.

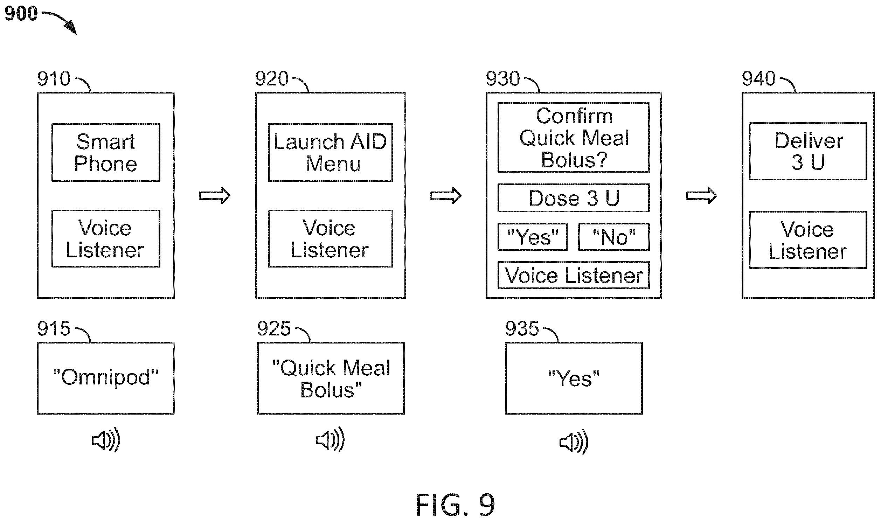

FIG. 9 illustrates an example voice control progression for causing an AID system to deliver a quick meal bolus according to the disclosed subject matter.

Similar to the voice control action sequences 601, 700 and 800, the voice control action sequence 900 begins at 910 with portable electronic device with an installed voice control application. As an initial mode, the voice control application may be in an active listening mode. In response to detecting the keyword "Omnipod" (915) at 910, the voice control application may be fully activated and begin listening for more complex commands other than keywords. In response to the detected keyword at 915, the voice control application may launch an AID menu, and the voice listener function remains active at 920 and detects the voice command phrase "Quick Meal Bolus," (925). The quick meal bolus delivers a nominal meal insulin bolus, which can be written as:

$$Meal\ Insulin\ Bolus\ Dose = x\%\ (Total\ Daily\ Insulin) + (BG - (Setpoint + Elevation))/$$

$$CorrectionFactor - IOB \quad (Equation\ 1)$$

Where, x may vary from 3% to 10% for example, and BG is a latest blood glucose measurement value. In this operational example as shown in FIG. 9, a total daily insulin (TDI) of 60U and the blood glucose measurement value (BG) is equal to setpoint + Elevation, and x = 5%, IOB = 0 U results in a quick meal bolus of 3 U of insulin for delivery.

At 930, the voice control application may present the information on the display device of the controller, and, in addition or alternatively, cause a speech synthesizer (not shown in this example) to output a confirmation request message. A confirmation request message (e.g., "Confirm Quick Meal Bolus of 3 Units") for delivery of 3 U of insulin may be output to graphical user interface on the display device, output to a speaker as an audio output, or both. In response to the presentation of the confirmation request message, the user may respond by selecting either the "Yes" button or the "No" button via an input to the graphical user interface, or by responding verbally with a "Yes" or a "No." After confirmation by the input of "YES" at 935, a bolus of 3 U is delivered. At 940, the voice control application may cause the presentation on a display, as an audio output from a speaker, or both that a bolus of 3 U has been delivered. The voice control application may return to an active listening mode.

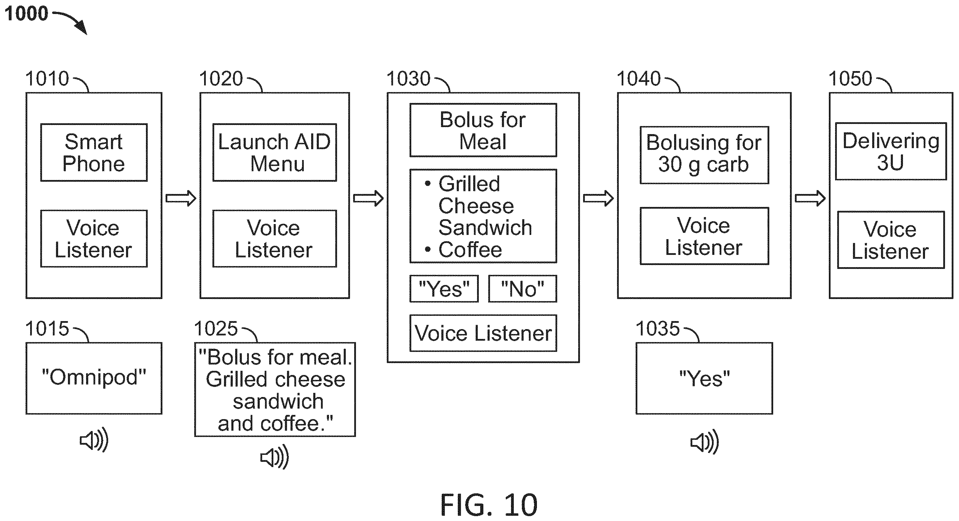

FIG. 10 illustrates an example voice control progression for causing an AID system to deliver a bolus based on a meal description according to the disclosed subject matter. Similar to the voice control action sequence 601 of FIG.

6, yet another embodiment may include the use of food descriptions for which an equivalent insulin dose is recommended.

In this example, the voice control action sequence 1000 may begin at 1010 with a portable electronic device with an installed voice control application. As an initial mode, the voice control application may be in the active listening mode as explained in earlier examples. In response to detecting the keyword "Omnipod" (1015) at 1010, the voice control application may be fully activated and begin listening for more complex commands other than keywords. In response to the detected keyword at 1015, the voice control application may launch an AID menu, and the voice listener function remains active at 1020 and detects the voice command phrase "Bolus for meal. Grilled cheese and Coffee," (1025). The voice control application using the previously discussed voice recognition functions causes a routine, or routines, such as a Meal Bolus Menu, to be called.

For example, a user may verbally describe the food being eaten to compensate with a bolus. As an example, the voice control application may detect a conversational statement of "Bolus for meal. Grilled cheese and Coffee." The detected conversational statement may be parsed by an NLU, and the food components may be identified and output as a recognition result. At 1030, the voice control application may populate a graphical user interface with the identified food components, such as "grilled cheese sandwich" and "coffee," and generate a confirmation request by presenting a "Yes" button and a "No" button. In addition, the voice control application may cause the output of the identified food components via a speaker as well as the confirmation request (e.g., "Please confirm meal of Grilled cheese sandwich and coffee. Yes or No"). In response to detecting a "Yes" (1035) response to the confirmation request, the voice control application may interpret the detected "Yes" as a user's confirmation of the identified food components.

Alternatively, if the identified food components are incorrect based on the detection of a "NO" response instead of a "Yes," the voice control application may return to 1020 and generate a request via the speaker, the graphical user interface, or both to either re-state verbally the description of the food being eaten or enter the description into the graphical user interface.

Based on the user's confirmation (i.e., receipt of the "Yes" input) of the identified food components, the voice control application may cause communication circuitry of the portable electronic device or the wearable drug delivery device to query a food database for macronutrient information or carbohydrate information of the identified food components (e.g., grilled cheese sandwich and coffee). The macronutrient information may include an amount of protein, an amount of fat, and an amount of carbohydrates as identified food components. The carbohydrate information of the identified food components may be a total number of carbohydrates for the aggregate of the identified food components (i.e., both the grilled cheese sandwich and the coffee). The voice control application may be operable to provide information to the AID application so the AID application may take a corresponding insulin delivery action. In the FIG. 10 example, the information provided to the AID application may be a total number of carbohydrates for use in calculating a bolus dosage to compensate for the ingested meal. Upon receipt of the carbohydrate (or macronutrient) information, the voice control application may utilize the carbohydrate (or macronutrient) information and an insulin-to-carbohydrate ratio (ICR) of the user, as well as any correction terms to calculate a bolus dosage. The voice control application, at 1040, may generate a status update, such as "Bolusing for 30 g of carbs" or the like that is output to the graphical user interface on the display device, output to a speaker as an audio output, or both. Unless a user input indicating that the bolus should not be delivered is received, the voice control application may pass the number of grams of carbohydrates to the AID application for calculation of the bolus dosage. At 1050, the voice control application may receive from the AID application the bolus dosage to be delivered. In this example, the bolus dosage is 3U. The voice control application may cause the presentation of a notification, such as "Delivering 3U" as either a visual notification, an audio notification or both. The voice control application may continue to monitor for any verbal inputs. If no verbal inputs or inputs to the graphical user interface are received, the AID application may cause delivery of the 3U of insulin.

In this example, the NLU application or applet parses the phrase and populates the Meal Bolus menu with IOB (Insulin on Board), the blood glucose value from the CGM as well as the correct over blood glucose terms and the correction factor. The voice control application may return to an active listening mode.

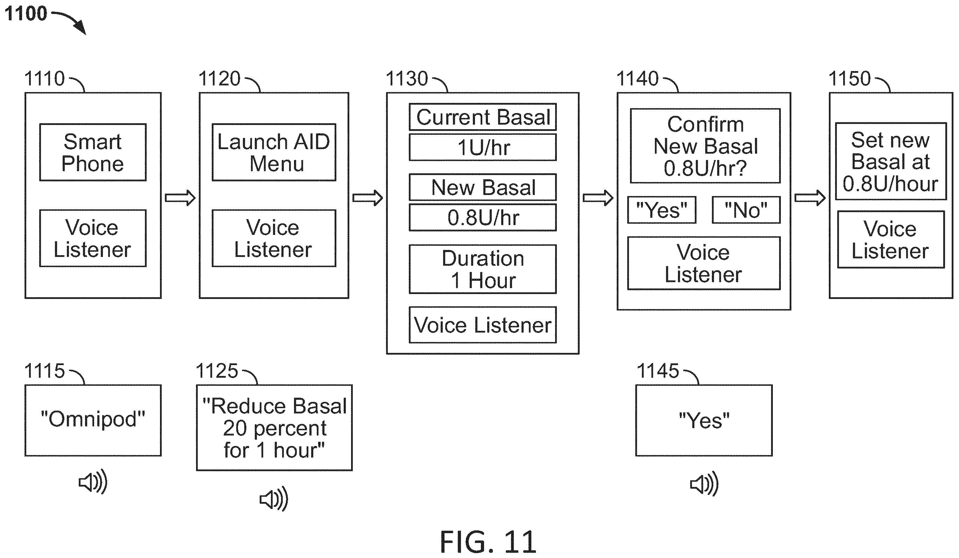

FIG. 11 illustrates an example voice control progression for causing an AID system to deliver basal insulin at a reduced basal rate according to the disclosed subject matter.

A temporary basal setting may also be set using voice control. The basal setting is used to deliver insulin needed for basic metabolic activities in the human body. Reduction in basal insulin might be needed during exercise to reduce the possibility of hypoglycemia. Temporary increases or decreases in basal insulin might be needed at other times as well to adjust to changing physiological needs of the body. In this voice command sequence example of FIG. 11, the user is requesting the basal rate to be reduced by 20% for 1 hour.

The voice control action sequence 1100 may begin at 1110 with portable electronic device with an installed voice control application. As an initial mode, the voice control application may be in an active listening mode. In response to detecting the keyword "Omnipod" (1115) at 1110, the voice control application may be fully activated and begin listening for more complex commands other than keywords. In response to the detected keyword at 1115, the voice control application may launch an AID menu, and the voice listener function remains active at 1120 and detects the voice command phrase "Reduce Basal 20 percent for 1 hour," (1125). The voice control application using the previously discussed voice recognition functions to recognize the voice command phrase and call a routine, or routines, of the voice control application, such as a basal setting change menu routine, based on the recognized voice command phrase.

For example, an NLU of the voice control application may parse the voice command phrase and populate the basal setting change menu. As shown at 1130, for example the NLU of the voice control application may populate fields of the basal setting change menu graphical user interface. The current basal rate is shown and the new basal rate at 80% of the current rate is shown. For example, the basal setting change menu graphical user interface may present a current basal rate (i.e., 1 Unit per hour (1 U/hr)), a new basal rate (i.e., 0.8 U/hr) and a duration (i.e., 1 hour) of an amount of time that the new basal rate will be delivered before reverting back to the previous setting of the current basal rate. The 1 -hour duration for the new basal rate may be a default setting obtained from the AID application. The duration may be a tunable parameter that may be changed by the user via a voice command, or automatically by the AID application. For example, the AID application may set the duration based on a user history (e.g., histories of CGM values, insulin delivery history, or the like), or other parameters. Also, at 1130, the voice control application may continue to monitor for any inputs (verbal, tactile, or both).

At 1140, the voice control application may present a confirmation menu on the graphical user interface that requests confirmation of the change from the current basal rate setting to the new basal rate setting. For example, the graphical user interface may present the message "Confirm New Basal 0.8 U/hr?" with a "Yes" input button and a "No" input button. In addition, the voice control application is in an active listening mode listening for either a "Yes" or "No" verbal input.

In response to the voice command of "Yes" (1145), the voice control application may cause the presentation of a message of "Set new basal (rate) at 0.8 U/hr" at 1150. At 1150, the voice control application may indicate to the AID application that the reduced basal rate has been confirmed. In response to the confirmation indication from the voice control application, the AID application may generate a signal that causes the wearable drug delivery device to begin delivering insulin at the basal rate of 0.8 U/hr for the set duration of 1 hour. The voice control application may return to an active listening mode.

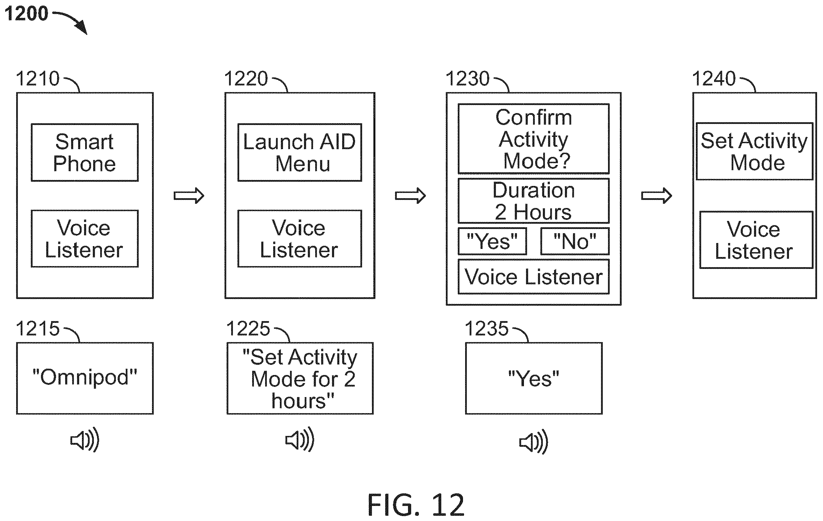

FIG. 12 illustrates an example voice control progression for causing an AID system to set an activity mode of an AID algorithm according to the disclosed subject matter.

The "activity mode" may be an operating mode of the AID application that considers the fact that the user may be exercising and that the increased physical activity may have an effect on the user's blood glucose measurements, insulin metabolism, and other physiological effects. The activity mode in an AID system may take actions such as changing one or more settings that reduce the risk of hypoglycemia during exercise. When in the activity mode, the AID application may change a number of parameters. For example, while in activity mode, the AID application may modify the target setpoint elevation so that the blood glucose level is maintained at a higher value during exercise and the insulin needs are reduced.

In this example, the voice control action sequence 1200 may begin at 1210 with portable electronic device with an installed voice control application. As an initial mode, the voice control application may be in an active listening mode. In response to detecting the keyword "Omnipod" (1215) at 1210, the voice control application may be fully activated and begin listening for more complex commands other than keywords. In response to the detected keyword at 1215, the voice control application may launch an AID menu, and the voice listener function of the voice control application remains active at 1220 and detects the voice command phrase "Set Activity Mode for 2 hours," (1225). The voice control application using the previously discussed voice recognition functions to recognize the voice command phrase and call a routine, or routines, of the voice control application, such as a mode change routine or the like, based on the recognized voice command phrase.

For example, an NLU of the voice control application may parse the voice command phrase and populate a graphical user interface for a mode change. As shown at 1230, for example, the NLU of the voice control application may populate fields of the mode change graphical user interface. For example, the voice control application may cause the graphical user to be populated with a request to confirm the change to the Activity Mode and a duration field for a duration of 2 hours. The 2-hour Activity Mode duration for the Activity Mode may be a default setting obtained from the AID application. The duration may be a tunable parameter that may be changed by the user or by the AID application. For example, the AID application may set the duration based on a user history of physical activities, such as time at the gym, a bicycle ride, or the like. Also, at 1230, the voice control application may continue to monitor for any inputs (verbal, tactile, or both).

At 1230, the voice control application may present a confirmation request screen on the graphical user interface that requests confirmation of the change from the current basal rate setting to the new basal rate setting. For example, the graphical user interface may present a message to "Confirm Activity Mode?" with "Duration 2 hours" and a "Yes" input button and a "No" input button. In addition, the voice control application is in an active listening mode listening for either a "Yes" or "No" verbal input in response to the confirmation request.

In response to the voice command of "Yes" (1235), the voice control application may cause the presentation of a message of "Set Activity Mode" at 1240. Additionally, or alternatively, in this and in each of the other embodiments, the voice control application may cause the wearable drug delivery device to vibrate to confirm receipt of the confirmation or of the voice command. For example, if a command or confirmation is successfully received, the wearable medical device may vibrate three short times; and if a voice command or confirmation is not successfully received, the wearable medical device may vibrate once. By way of further example, the wearable drug delivery device may vibrate upon recognizing that the user has used a wake-up keyword or phrase (such as "Hey Omnipod"). In addition, at 1240, the voice control application may indicate to the AID application that the setting of the activity mode has been confirmed. The voice control application may return to an active listening mode.

Other functions may also be set via voice control using commands such as "wearable drug delivery device activation," "wearable drug delivery device deactivation" (for removing the wearable drug delivery device), "querying bolus history," "query blood glucose history," "show current basal rate settings," "show current set point settings," "show current insulin to carbohydrate ratio settings," "show current correction bolus settings," or the like.

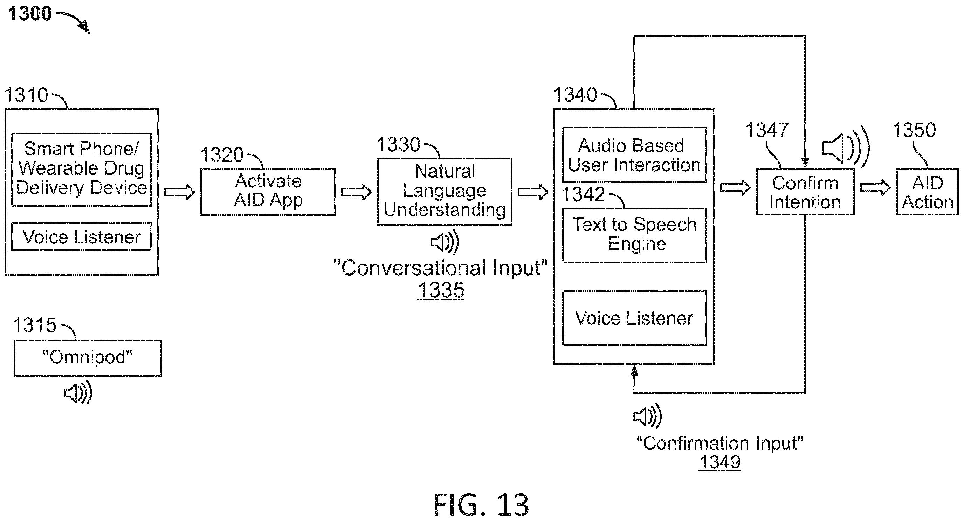

FIG. 13 illustrates an exemplary voice control progression for causing an AID system to receive confirmation of AID application action according to the disclosed subject matter. In this example, the voice control action sequence 1300 may begin at 1310 with a portable electronic device with an installed voice control application and an AID application (shown in other examples, such as FIG. 2). It is envisioned that portable electronic device 1310 may be either a smart phone or a wearable drug delivery device (that is adhered to the skin of a user). As an initial mode, the voice control application may be in an active listening mode. When in the active listening mode, at 1310, the voice control application may be listening individual inputs of a set of activation keywords (e.g., "Omnipod," "wearable drug delivery device," custom words, "insulin," or the like). fully activated and begin listening for more complex verbal commands other than keywords. In response to the detected activation keyword (e.g., "Omnipod") at 1315, the voice control application may cause the AID application to be activated (1320). In addition, the voice control application may call a natural language understanding (NLU) module at 1330. The NLU may be operable to understand verbal inputs that are more complex than the collection of keywords and phrases described in the earlier examples of FIGs. 9-12. At 1340, the NLU processing may enable an audio-based user interaction function during which the NLU is operable to determine user intention based on a conversational input/dialog (1335). For example, a user may speak a conversational input (1335) that may be a command for the AID application to take some action. The NLU unit may recognize the conversational input and provide a signal representation of the recognized conversational input to the voice control application. The voice control application may utilize a text-to-speech engine 1342 to generate an audio output or a visual output that is a request for confirmation of the intent of the conversational input 1335. At 1347, the voice control application may pause a predetermined amount of time to receive a confirmation input 1349. After the intent of the conversational input 1335 is confirmed by confirmation input 1349, the voice control application may forward a command, or commands, based on the recognized intent to the AID application. Using the forwarded commands, the AID application may take the appropriate action (1350). The voice control action sequence 1300 is an interaction that takes place hands free which can be very advantageous to a user that is engaged in physical activity (e.g., bicycle/running, baseball, walking a dog, paddleboarding, or the like), activities where the hands are engaged (e.g., carrying a bag or suitcase, driving, cooking, working, or the like), or for patients with physical limitations including diminished dexterity, and the like.

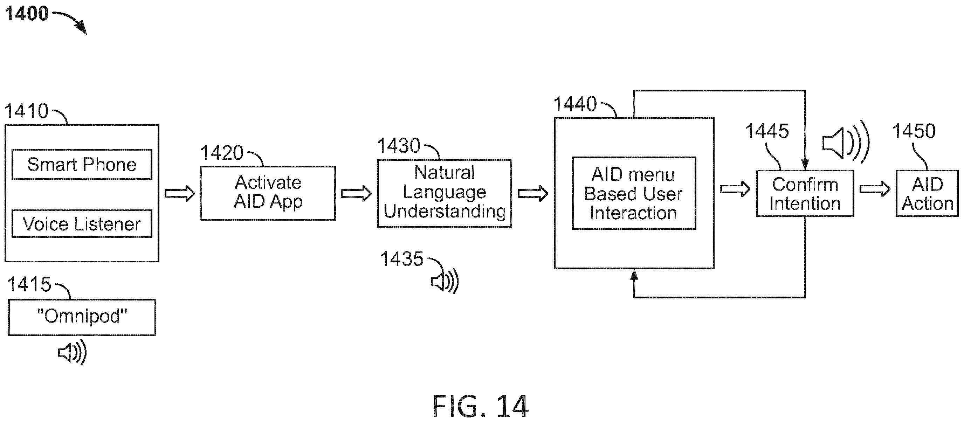

FIG. 14 illustrates an exemplary voice control progression for interacting with AID system control menu according to the disclosed subject matter.

In this embodiment, which is different from the embodiment of FIG. 5, both visual and audio-based confirmations may possibly converge on user intent. This convergence offers a voice control progression with increased flexibility for the user and a reliable capture of user intent. For example, the voice control action sequence 1400 may begin at 1410 with a portable electronic device with an installed voice control application and an AID application. As an initial mode, the voice control application may be in an active listening mode. In active listening mode, the voice control application may be listening for a subset of commands or keywords that indicate the user wishes to interact with the voice control application via voice commands. In response to the detected keyword (e.g., "Omnipod") such as at 1415, the voice control application may cause the AID application to be activated and begin listening for more complex verbal commands other than a subset of keywords (1420). In addition, the voice control application may call a natural language understanding (NLU) module at 1430. The NLU may be operable to understand verbal inputs that are more complex than the collection of keywords and phrases described in the earlier examples of FIGs. 9-12. For example, at 1440, the NLU processing may enable an audio-based user interaction function to present on the display of a controller an AID menu. The AID menu may be a graphical user interface that is navigable using audio command, such as 1435, as well as touch inputs. The audio command may be a keyword or a string of keywords, a conversational phrase, or the like.

Upon receipt of the audio command 1435, the NLU of the voice control application may interpret the audio command 1435 and generate an interpretation signal representing the interpretation of the audio command. The voice control application may cause the presentation of fields of a menu of AID actions (1440) based on the interpretation signal as well as cause a speech generation algorithm, such as a speech-to-text algorithm, to generate an audio output that corresponds to the presented field of the AID menu. The AID menu may list a number of AID actions from which a user may select to implement. For example, a processor may present on a display screen the menu of AID actions populated with a number of AID actions that may be selected for implementation. The voice control application may wait at 1445 for a user interaction. The processor may receive a selection of one of the presented AID actions. After a period of time or receipt of a verbal or tactile indication that the user has completed their selections.

Upon receipt of a user confirm at 1445, such as receipt of a keyword, such as "Yes," "Confirm," "Proceed," or the like, the voice control application may enable the AID application to take action (1450) corresponding to the audio command 1435. Of course, if the interpretation is incorrect, the user may request a "Re-do," "Go back" or "No" and the voice control application may receive a request for the user to restate their audio command. The user can get to see the choices that have been made. Alternatively, or in addition, at 1445, the AID menu presented on the controller may be enabled to present a number of simple tabs, such as "yes" or "no," that enable a user to select one of the simple tabs to confirm intent and cause the AID application to deliver the AID action. In this example, the voice control application is operable to provide both visual and audio confirmation.

As shown in each of the foregoing examples, interacting with the AID system via audio commands is particularly useful and advantageous when the user is otherwise physically engaged, such as carrying packages, an umbrella, participating in a physical activity, such as cycling, kayaking, washing dishes, gardening, or the like. For example, the bicycling user may request that an AID menu for setting blood glucose target set points be presented, and using voice commands may navigate the AID menu with options for changing the present day's current blood glucose target set points, or preparing the AID system for a transition to a resupply of insulin, setting the "activity mode," or the like.

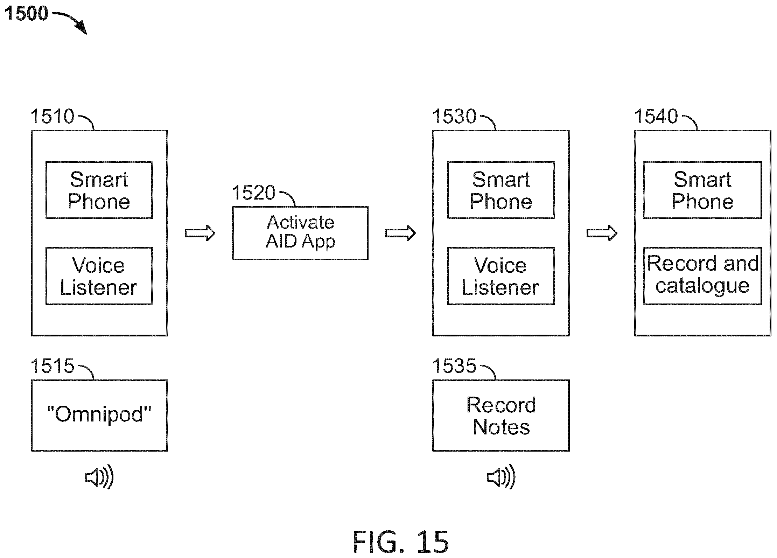

FIG. 15 illustrates an exemplary voice control progression for causing an AID system to record annotations related the AID system according to the disclosed subject matter.

In this embodiment, the user is provided with the option to record voice annotations to describe their experiences through the day, post meals, at bedtime, upon wake up and the like. For example, the voice control action sequence 1500 may begin at 1510 with a portable electronic device with an installed voice control application and an AID application. As an initial mode, the voice control application may be in an active listening mode. In active listening mode, the voice control application may be listening for a subset of commands or keywords that indicate the user wishes to interact with the voice control application via voice commands. In response to the detected keyword (e.g., "Omnipod") such as at 1515, the voice control application may cause the AID application to be activated (1520). After receipt of the detected keyword, the voice control application may be fully activated and begin listening for more complex verbal commands or a full set of keywords, or as in this example, annotations. At 1530, the voice control application may utilize a natural language understanding (NLU) module to understand the spoken annotations. For example, the user may speak notes (1535) that the user wishes to be associated with a period of time or an event (e.g., physical activity or stressful situation) that may give context to data obtained by the drug delivery system during the period of time or the event. The annotations, for example, may be related to related to a user impression of a period of time related to a treatment program of the user, such as "feeling fine this morning," "slept well last night," "slept fitfully last night," "feeling hyperglycemic after a heavy meal," "feeling great after running 2 miles," or the like. The period of time may be, for example, "this morning" (e.g., 6 am to 11 am), "last night" (e.g., 9 pm to 6 am), "after running 2 miles" (e.g., the present time minus approximately 20 minutes or based on an amount of time from a fitness or heartrate monitor), or the like. Using the recognition results from the NLU, the recognized recordings, at 1540, may be stored and catalogued in a memory. The AID application may be operable to generate insights and personalization of the AID application settings and parameters, blood glucose histories, drug delivery histories, and the like.

In an example, sound chip circuitry or a voice control application may be operable to receive a verbal annotation related to a user impression of a period of time related to a treatment program of the user. A processor may categorize the verbal annotation based on an output of a natural language understanding module. A digital representation of the verbal annotation may be stored in a memory based on the categorization of the verbal annotation. The processor may use the stored digital representation of the verbal annotation to generate a treatment program recommendation for output to the user.

While the previous examples of FIGs. 5-15 show and describe implementations in a smartphone, this was done for ease of discussion. It is also envisioned that the foregoing voice control application sequences may, in addition to the smartphone, or as an alternative to the smartphone, be implemented on a wearable drug delivery device, such as wearable drug delivery device 102, that is equipped with the voice control application such as 188 of FIG. 1, or equipped with a voice control circuitry/application 238 and an NLU module 237 of FIG. 2.

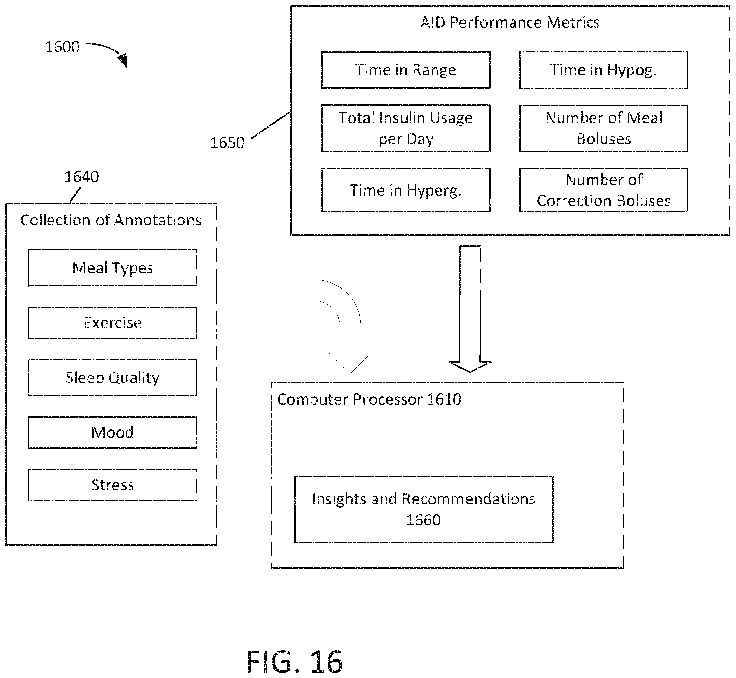

FIG. 16 illustrates an exemplary process for evaluating the annotations recorded according to FIG. 15 with respect to metrics affecting a user's insulin treatment program according to the disclosed subject matter. The process 1600 is a data analysis process by which the annotations in the collection of annotations 1640 may be analyzed by a computer processor 1610 with respect to the AID performance metrics 1650 to provide insights and recommendations 1660 to the user's insulin treatment plan. For example, the computer processor 1610 may be specifically programmed to evaluate and process the analysis performed by the voice control application and/or the AID application to provide both coarse and fine granular insights to effects on the user's insulin treatment plan.

A memory may store a collection of annotations 1640 that have been obtained as described with reference to FIG. 15. The memory may be resident on the wearable drug delivery device, the portable electronic device (e.g., smartphone or controller), or an external storage device, such as in a server or a cloud platform, which may be accessible by the portable electronic device and/or the wearable drug delivery device. The voice control application may store the collection of annotations 1640 in a memory and in files that may be categorized in different categories relevant to the user's diabetes treatment plan. For example, the collection of annotations 1640 may be categorized in categories such as meal types, exercise, sleep quality, mood, and stress. One purpose for capturing the annotations is provide the user with additional qualitative information that may not be captured by the evaluation of the AID performance metrics alone.

Combining the collection of annotations 1640 obtained, via the voice control progression example of FIG. 15, with stored AID performance metrics 1650 obtained by monitoring user history can provide otherwise undetectable insights to the user and/or the user's healthcare provider. The monitored user history can, for example, include blood glucose measurement history, ketone measurement history, drug delivery history, total daily insulin values, insulin on-board calculations and the like. Examples of the AID performance metrics may include an amount of time in range, a total insulin usage per day, an amount of time in hyperglycemic range (e.g., blood glucose measurement values greater than 180 mg/dL), an amount of time in hypoglycemic range (e.g., blood glucose measurement values less than 70 mg/dL), a number of meal boluses, a number of correction boluses, or the like. The AID performance metrics 1650 may be acquired by the AID application and stored in a memory.

In an operational example, the computer processor 1610 executing the AID application may access the collection of annotations 1640 and the AID performance metrics 1650. The processor may analyze the collection of annotations 1640 and the AID performance metrics 1650. Based on the analysis, the processor executing the AID application may determine that a particular type of meal resulted in the user experiencing hyperglycemia. The insights and recommendations 1660 provided by computer processor 1610 may be used by the AID application to provide a recommendation for the user to eat that particular type of meal sparingly. Alternatively, or in addition, the AID application using the insights and recommendations 1660 from the computer processor 1610 may provide a better bolusing recommendation for that meal (e.g., a recommendation for a specific bolus timing and a bolus dosage quantity). In a further example of the benefits of the insights and recommendations 1660, the AID application may be operable to show blood glucose variations around exercise (during and after) to the user to enable the user to better understand how their blood glucose changes with a particular type and duration of exercise. Based on blood glucose outcomes, the AID application may be operable to provide recommendations for insulin delivery adjustments. In addition, the effects of too little sleep and/or too much stress may be revealed to the user.

As exemplary insights of the insights and recommendations 1660, a typical meal may be tracked by the post prandial blood glucose excursion. For example, if the post prandial (e.g., 3-hour period after meal) blood glucose (BG) is above 180 mg/dl for a substantial time (for example 30% in 3-hour period), then either the bolus was inadequate, or the timing was incorrect, or the glycemic index of the food or macronutrient of the food was not matched to the bolus. A recommendation may be made to alter bolus timing, bolus amount, or bolus delivery profile (such as, for example, splitting the bolus into an upfront delivery and a later delivery). Another insight or recommendation may be to avoid this food as the blood glucose outcome was poor. An exemplary insight for exercise might track the blood glucose during and after exercise for the next 3 hours. If either after or during exercise, any hypoglycemia occurs (BG < 70 mg/dl), then the insulin delivery needs to be reduced. Feedback to the user may include turning on an activity mode of the AID application earlier, voice commands for basal reduction during and post exercise. Alternatively, another feedback insight may be to ingest some carbohydrates before/during exercise.

While insulin is referred to often as the therapeutic drug delivered by the wearable drug delivery device in the above discussion, the insulin may be replaced by other drugs analogous to insulin, glucagon-like peptide-1 (GLP-1), pramlintide, glucagon, co-formulations of two or more of GLP-1, pramlintide, and insulin; as well as pain relief drugs, such as opioids or narcotics (e.g., morphine, or the like), methadone, arthritis drugs, hormones, such as estrogen and testosterone, blood pressure medicines, chemotherapy drugs, fertility drugs, or the like.. Of course, inputs to the above equations may be from sensors other than a continuous blood glucose monitor, such as a fingertip glucose sensor but similar equations and actions may be taken.

Software related implementations of the techniques described herein, such as the examples described with reference to FIGs. 1-16 may include, but are not limited to, firmware, application specific software, applet, or any other type of computer readable instructions that may be executed by one or more processors. The computer readable instructions may be provided via non-transitory computer-readable media. The various elements of the processes described with reference to the figures may be implemented in devices, apparatuses or systems that may include various hardware elements, software elements, or a combination of both. Examples of hardware elements that may be the basis for a computer processor may include structural members, logic devices, components, processors, microprocessors, circuits, processors, circuit elements (e.g., transistors, resistors, capacitors, inductors, and so forth), integrated circuits, application specific integrated circuits (ASIC), programmable logic devices (PLD), digital signal processors (DSP), field programmable gate array (FPGA), memory units, logic gates, registers, semiconductor device, chips, microchips, chip sets, and so forth. Examples of software elements may include software components, programs, applications, computer programs, application programs, system programs, software development programs, machine programs, operating system software, middleware, firmware, software modules, routines, subroutines, functions, processes, procedures, software interfaces, application program interfaces (API), instruction sets, computing code, computer code, code segments, computer code segments, words, values, symbols, or any combination thereof.. The computer programs of the present disclosure may be for example preinstalled on, or downloaded to the medicament delivery system, medicament delivery device, management device, fluid delivery device, e.g. their storage.

Some examples of the disclosed device or processes may be implemented, for example, using a storage medium, a computer-readable medium, or an article of manufacture which may store an instruction or a set of instructions that, if executed by a machine (i.e., processor or controller), may cause the machine to perform a method and/or operation in accordance with examples of the disclosure. Such a machine may include, for example, any suitable processing platform, computing platform, computing device, processing device, computing system, processing system, computer, processor, or the like, and may be implemented using any suitable combination of hardware and/or software. The computer-readable medium or article may include, for example, any suitable type of memory unit, memory, memory article, memory medium, storage device, storage article, storage medium and/or storage unit, for example, memory (including non-transitory memory), removable or non-removable media, erasable or non-erasable media, writeable or re-writeable media, digital or analog media, hard disk, floppy disk, Compact Disk Read Only Memory (CD-ROM), Compact Disk Recordable (CD-R), Compact Disk Rewriteable (CD-RW), optical disk, magnetic media, magneto-optical media, removable memory cards or disks, various types of Digital Versatile Disk (DVD), or the like. The instructions may include any suitable type of code, such as source code, compiled code, interpreted code, executable code, static code, dynamic code, encrypted code, programming code, and the like, implemented using any suitable high-level, low-level, object-oriented, visual, compiled and/or interpreted programming language. The non-transitory computer readable medium embodied programming code may cause a processor when executing the programming code to perform functions, such as those described herein.

Certain examples of the present disclosure were described above. It is, however, expressly noted that the present disclosure is not limited to those examples, but rather the intention is that additions and modifications to what was expressly described herein are also included within the scope of the disclosed examples. Moreover, it is to be understood that the features of the various examples described herein were not mutually exclusive and may exist in various combinations and permutations, even if such combinations or permutations were not made express herein, without departing from the spirit and scope of the disclosed examples. In fact, variations, modifications, and other implementations of what was described herein will occur to those of ordinary skill in the art without departing from the spirit and the scope of the disclosed examples. As such, the disclosed examples are not to be defined only by the preceding illustrative description.

Program aspects of the technology may be thought of as "products" or "articles of manufacture" typically in the form of executable code and/or associated data that is carried on or embodied in a type of non-transitory, machine readable medium. Storage type media include any or all of the tangible memory of the computers, processors or the like, or associated modules thereof, such as various semiconductor memories, tape drives, disk drives and the like, which may provide non-transitory storage at any time for the software programming. It is emphasized that the Abstract of the Disclosure is provided to allow a reader to quickly ascertain the nature of the technical disclosure. It is submitted with the understanding that it will not be used to interpret or limit the scope or meaning of the claims. In addition, in the foregoing Detailed Description, various features are grouped together in a single example for streamlining the disclosure. This method of disclosure is not to be interpreted as reflecting an intention that the claimed examples require more features than are expressly recited in each claim. Rather, as the following claims reflect, novel subject matter lies in less than all features of a single disclosed example. Thus, the following claims are hereby incorporated into the Detailed Description, with each claim standing on its own as a separate example. In the appended claims, the terms "including" and "in which" are used as the plain-English equivalents of the respective terms "comprising" and "wherein," respectively. Moreover, the terms "first," "second," "third," and so forth, are used merely as labels and are not intended to impose numerical requirements on their objects.

The foregoing description of examples has been presented for the purposes of illustration and description. It is not intended to be exhaustive or to limit the present disclosure to the precise forms disclosed. Many modifications and variations are possible considering this disclosure. It is intended that the scope of the present disclosure be limited not by this detailed description, but rather by the claims appended hereto. Future filed applications claiming priority to this application may claim the disclosed subject matter in a different manner and may generally include any set of one or more features as variously disclosed or otherwise demonstrated herein.

Although the present invention is defined in the attached claims, it should be understood that the present invention can also (alternatively) be defined in accordance with the following embodiments:

   1. A wearable drug delivery device, comprising:

a processor operable to execute programming instructions;
communication circuitry coupled to the processor and operable to receive and transmit information;
sound chip circuitry including a recording memory, a microphone, a speaker, a processor, and a voice control application, wherein the recording memory, the microphone, the speaker, and the voice control circuitry are coupled to one another and the recording memory is operable to store a plurality of audio clips; and
a memory coupled to the processor and operable to store the programming instructions and, wherein the sound chip circuitry is operable to:

identify a specific audio clip from the plurality of audio clips stored in the recording memory; and
output the specific audio clip via the speaker.

2. The wearable drug delivery device of embodiment 1, wherein the sound chip is further operable, to:

determine a time of day; and
use the determined time of day when identifying the specific audio clip from the plurality of audio clips.

3. The wearable drug delivery device of embodiment 1, wherein the sound chip is further operable, to:

determine a location of a controller associated with the voice control application; and
use the determined location of the controller when identifying the specific audio clip from the plurality of audio clips.

4. The wearable drug delivery device of embodiment 1, wherein the sound chip is further operable, to:

determine a location of a controller associated with the voice control application;
determine a time of day; and
use the determined time of day and the determined location of the controller when identifying the specific audio clip from the plurality of audio clips.

5. The wearable drug delivery device of embodiment 1, wherein the sound chip is further operable, to:

receive an indication that a battery of the controller is being recharged; and
in response to the received indication, begin the identifying of the specific audio clip from the plurality of audio clips.

6. The wearable drug delivery device of embodiment 1, wherein the sound chip, prior to identifying the specific audio clip from the plurality of audio clips, is further operable, to:

receive an indication that a user selected a record audio function;
record audio in response to the received indication of selection of the record audio function; and
store the recorded audio in the recording memory with the plurality of audio clips.

7. The wearable drug delivery device of embodiment 1, wherein the sound chip, prior to identifying the specific audio clip from the plurality of audio clips, is further operable, to:

receive an indication that a user selected a record audio function;
record audio in response to the received indication of selection of the record audio function; and
store the recorded audio in the recording memory with the plurality of audio clips.

8. The wearable drug delivery device of embodiment 1, wherein the sound chip is further operable, to:

receive an unpackaging indication that a package containing the wearable drug delivery device has been opened; and
use the unpackaging indication to identify the specific audio clip from the plurality of audio clips, wherein the identified specific audio clip includes instructions related to initializing the wearable drug delivery device for use by the user.

9. A wearable drug delivery device, comprising:

a processor operable to execute programming instructions;
communication circuitry coupled to the processor and operable to receive and transmit information;
sound chip circuitry including a microphone, a speaker, and voice control circuitry, wherein the microphone, the speaker and the voice control circuitry are coupled to one another; and
a memory coupled to the processor and operable to store the programming instructions, wherein the sound chip circuitry is operable to:

receive a voice command indicative of an insulin delivery action to be taken by an automated insulin delivery application;
determine the received voice command corresponds to an identified insulin delivery action to be taken by the automated insulin delivery application;
output a confirmation request;
monitor for receipt of an input that is a confirmation response to the confirmation request; and
in response to receipt of the confirmation response, enable the insulin delivery action to be taken by the automated insulin delivery application.

10. The wearable drug delivery device of embodiment 9, wherein the sound chip circuitry, prior to receiving the voice command indicative of the insulin delivery action, is operable to:

determine a verbal input is an activation keyword; and
in response to the activation keyword, the voice control circuitry switches from an active listening mode to a full activation mode, wherein the full activation mode
includes recognition of keywords that form the voice command indicative of an insulin delivery action.

11. The wearable drug delivery device of embodiment 9, wherein the sound chip circuitry is further operable to:
receive a plurality of signals within the current period of time, wherein each signal of the plurality of signals includes at least the discrete amount of insulin to be delivered by the wearable drug delivery device.

12. The wearable drug delivery device of embodiment 9, wherein the confirmation request indicates the action to be taken by the automated insulin delivery application and sets the voice control application for receipt of a confirmation input related to the action to be taken by the automated insulin delivery application.

13. The wearable drug delivery device of embodiment 9, wherein the sound chip circuitry further comprises a natural language understanding module, and the sound chip circuitry is further operable to:
call the natural language understanding module for further interpretation and recognition of terms and phrases that form keywords of the voice command indicative of the insulin delivery action and inputs.

14. The wearable drug delivery device of embodiment 9, wherein the sound chip circuitry, when enabling the insulin delivery action to be taken by the automated insulin delivery application, is further operable to:

determine the received voice command corresponds to an identified insulin delivery action based on a recognition result; and
forward a signal representing the corresponding insulin delivery action to an AID application for execution.

15. A drug delivery system, comprising:

a processor operable to execute programming instructions;
communication circuitry coupled to the processor and operable to receive and transmit information;
sound chip circuitry including at least two of the following: a microphone, a speaker, a vibration device, or voice control circuitry; and
a memory coupled to the processor and operable to store the programming instructions, wherein the sound chip circuitry is operable to:

receive a voice command indicative of an insulin delivery action to be taken by an automated insulin delivery application;
determine the received voice command corresponds to an identified insulin delivery action to be taken by the automated insulin delivery application;
output a confirmation request;

monitor for receipt of an input that is a confirmation response to the confirmation request; and
in response to receipt of the confirmation response, enable the insulin delivery action to be taken by the automated insulin delivery application.

16. The drug delivery system of embodiment 15, wherein the voice control circuitry of the sound chip circuitry further comprises:
a sound chip processor and a voice control application executable by the sound chip processor.

17. The drug delivery system of embodiment 15, wherein the sound chip circuitry further comprises:
a text to speech engine operable to generate the confirmation request.

18. The drug delivery system of embodiment 15, further comprising:
a display device communicatively coupled to the processor, wherein the processor, prior to outputting the audio confirmation request message, is further operable to:
cause a display device to present the identified insulin delivery action in a graphical user interface.

19. The drug delivery system of embodiment 15, further comprises:

a display device communicatively coupled to the processor, and
wherein the received voice command indicative of an insulin delivery action to be taken is a presentation of a menu of AID actions, and the processor, prior to outputting the audio confirmation request message, is further operable to:

present, on the display device, a menu of AID actions populated with a plurality of AID actions that may be selected for implementation;
receive a selection of one of the presented AID actions.

20. The wearable drug delivery system of embodiment 15, wherein the processor is further operable to:

receive a verbal annotation related to a user impression of a period of time related to a treatment program of the user;
categorize the verbal annotation based on an output of a natural language understanding module;
store a digital representation of the verbal annotation in memory based on the categorization of the verbal annotation; and
use the stored digital representation of the verbal annotation to generate a treatment program recommendation for output to the user.

**Claims**

1. A wearable drug delivery system, comprising:

a processor operable to execute programming instructions;
communication circuitry coupled to the processor and operable to receive and transmit information;
sound chip circuitry including a recording memory, a speaker, and a processor, wherein the recording memory, and the speaker are coupled to one another and the recording memory is operable to store a plurality of audio clips; and
a memory coupled to the processor and operable to store the programming instructions and, wherein the sound chip circuitry is operable to:

identify a specific audio clip from the plurality of audio clips stored in the recording memory; and
output the specific audio clip via the speaker.

2. The wearable drug delivery system of claim 1, wherein the sound chip circuitry is further operable, to:

determine a time of day; and
use the determined time of day when identifying the specific audio clip from the plurality of audio clips.

3. The wearable drug delivery system of claim 1 or 2, wherein the sound chip circuitry is further operable, to:

   determine a location of a controller associated with the voice control application; and
   use the determined location of the controller when identifying the specific audio clip from the plurality of audio clips.

4. The wearable drug delivery system of any one of claims 1 to 3, wherein the sound chip circuitry is further operable, to:

   determine a location of a controller associated with the voice control application;
   determine a time of day; and
   use the determined time of day and the determined location of the controller when identifying the specific audio clip from the plurality of audio clips.

5. The wearable drug delivery system of any one of claims 1 to 4, wherein the sound chip circuitry is further operable, to:

   receive an indication that a battery of the controller is being recharged; and
   in response to the received indication, begin the identifying of the specific audio clip from the plurality of audio clips.

6. The wearable drug delivery system of any one of claims 1 to 5, wherein the sound chip circuitry furthermore includes a microphone and a voice control application, wherein the recording memory, the microphone, the speaker, and the voice control circuitry are coupled to one another and wherein the sound chip, prior to identifying the specific audio clip from the plurality of audio clips, is further operable, to:

   receive an indication that a user selected a record audio function;
   record audio in response to the received indication of selection of the record audio function; and
   store the recorded audio in the recording memory with the plurality of audio clips.

7. The wearable drug delivery system of any one of claims 1 to 6, wherein the sound chip circuitry, is comprised within a drug delivery device, wherein the drug delivery device comprises a reservoir and wherein the drug delivery device is operable based on control signals from the processor to expel the drugs, medications, or therapeutic agents from the reservoir, in particular wherein the drug delivery device is attached to the user's skin, in particular wherein the drug delivery device is attached to the user's skin by an adhesive pad..

8. The wearable drug delivery system of any one of claims 1 to 7, wherein the sound chip circuitry is further operable, to:

   receive an unpackaging indication that a package containing the wearable drug delivery device has been opened; and
   use the unpackaging indication to identify the specific audio clip from the plurality of audio clips, wherein the identified specific audio clip includes instructions related to initializing the wearable drug delivery device for use by the user.

9. A wearable drug delivery system, comprising:

   a processor operable to execute programming instructions;
   communication circuitry coupled to the processor and operable to receive and transmit information;
   sound chip circuitry including a microphone, and voice control circuitry, wherein the microphone, and the voice control circuitry are coupled to one another; and
   a memory coupled to the processor and operable to store the programming instructions,
   wherein the sound chip circuitry is operable to:

   receive a voice command indicative of an insulin delivery action to be taken by an automated insulin delivery application;
   determine the received voice command corresponds to an identified insulin delivery action to be taken by the automated insulin delivery application;
   enable the insulin delivery action to be taken by the automated insulin delivery application.

10. The wearable drug delivery system of claim 9, wherein the sound chip circuitry, prior to receiving the voice command indicative of the insulin delivery action, is operable to:

determine a verbal input is an activation keyword; and

in response to the activation keyword, the voice control circuitry switches from an active listening mode to a full activation mode, wherein the full activation mode includes recognition of keywords that form the voice command indicative of an insulin delivery action.

11. The wearable drug delivery system of claim 9 or 10, wherein the sound chip circuitry is further operable to after determining the received voice command corresponds to an identified insulin delivery action to be taken by the automated insulin delivery application:

output a confirmation request;

monitor for receipt of an input that is a confirmation response to the confirmation request; and

in response to receipt of the confirmation response;

enable the insulin delivery action to be taken by the automated insulin delivery application.

12. The wearable drug delivery system of any one of claims 9 to 11, wherein the confirmation request indicates the action to be taken by the automated insulin delivery application and sets the voice control application for receipt of a confirmation input related to the action to be taken by the automated insulin delivery application.

13. The wearable drug delivery system of any one of claims 9 to 12, wherein the sound chip circuitry further comprises a natural language understanding module, and the sound chip circuitry is further operable to:
call the natural language understanding module for further interpretation and recognition of terms and phrases that form keywords of the voice command indicative of the insulin delivery action and inputs.

14. The wearable drug delivery system of any one of claims 9 to 13, wherein the sound chip circuitry, when enabling the insulin delivery action to be taken by the automated insulin delivery application, is further operable to:

determine the received voice command corresponds to an identified insulin delivery action based on a recognition result; and

forward a signal representing the corresponding insulin delivery action to an AID application for execution.

15. The wearable drug delivery system of any one of claims 9 to 14,
wherein the sound chip circuitry, is comprised within a drug delivery device, wherein the drug delivery device comprises a reservoir and wherein the drug delivery device is operable based on control signals from the processor to expel the drugs, medications, or therapeutic agents from the reservoir, in particular wherein the drug delivery device is attached to the user's skin, in particular wherein the drug delivery device is attached to the user's skin by an adhesive pad.

16. The drug delivery system of any one of claims 9 to 15, wherein the voice control circuitry of the sound chip circuitry further comprises:
a sound chip processor and a voice control application executable by the sound chip processor.

17. The drug delivery system of any one of claims 10 to 16, wherein the sound chip circuitry further comprises:
a text to speech engine operable to generate the confirmation request in the form of an audio confirmation request.

18. The drug delivery system of claim 17, further comprising:
a display device communicatively coupled to the processor, wherein the processor, prior to outputting the audio confirmation request message, is further operable to:
cause a display device to present the identified insulin delivery action in a graphical user interface.

19. The drug delivery system of claim 15, further comprises:

a display device communicatively coupled to the processor, and

wherein the received voice command indicative of an insulin delivery action to be taken is a presentation of a menu of AID actions, and the processor, prior to outputting the audio confirmation request message, is further operable to:

present, on the display device, a menu of AID actions populated with a plurality of AID actions that may be selected for implementation;

receive a selection of one of the presented AID actions.

20. The wearable drug delivery system of claim 15, wherein the processor is further operable to:

receive a verbal annotation related to a user impression of a period of time related to a treatment program of the user;
categorize the verbal annotation based on an output of a natural language understanding module;
store a digital representation of the verbal annotation in memory based on the categorization of the verbal annotation; and
use the stored digital representation of the verbal annotation to generate a treatment program recommendation for output to the user.

FIG. 1

FIG. 2

FIG. 3

Portable Electronic
Device 410

Voice Control
Application 415

AUDIO MSG
SYN 416

Txt-Speech
418

Communication
Circuitry 412

Processor
414

441

Wearable Drug Delivery Device 420

Sound Chip 430

Microphone/
Speaker 432

Recording Memory
434

Power Source 436

Voice Control App
438

AID System 450

Communication
Circuitry 452

Processor
454

Couplings
456

442

Analyte Sensor 460

443

FIG. 4

EP 4 401 084 A2

FIG. 5

500

510 — Portable Electronic Device / Voice Listener Function

520 — Launch AID Menu / Voice Listener Function

530 — Populate AID Fields / Voice Listener Function

540 — Confirm Entry / Voice Listener Function

545 — If Not Confirmed

550 — AID Action / Voice Listener Function

600

Receive a voice command indicative of an insulin delivery action to be taken by an automated insulin delivery application    611

Determine the received voice command corresponds to an identified insulin delivery action to be taken by the automated insulin delivery application    621

Output a confirmation request    631

Monitor for receipt of an input that is a confirmation response to the confirmation request    641

In response to receipt of a confirmation response, enable the insulin delivery action to be taken by the automated insulin delivery application    651

FIG. 6A

FIG. 6B

**700**

**710** Smart Phone / Voice Listener

**720** Launch AID Menu / Voice Listener

**730**
| Carbs | ICR |
|-------|-----|
| 60g | 10 |
| IOB | CGM |
| 1U | 180 |

| Correct Over | Correction Factor |
|--------------|-------------------|
| 120 | 60 |

Voice Listener

**740** Confirm Bolus 6U? / "Yes" "No" / Voice Listener

**750** Deliver Bolus 6U / Voice Listener

**715** "Omnipod"

**725** "Bolus for Meal 60g Carb With Correction"

**745** "Yes"

FIG. 7

EP 4 401 084 A2

**800**

**810**

Smart Phone

Voice Listener

**815**

"Omnipod"

🔊

**820**

Launch AID Menu

Voice Listener

**825**

"Correction Bolus"

🔊

**830**

| CGM | IOB |
|---|---|
| 240 | 0.5U |

| Correct Over | Correction Factor |
|---|---|
| 120 | 60 |

Voice Listener

**840**

Confirm Bolus 1.5U?

"Yes"   "No"

Voice Listener

**845**

"Yes"

🔊

**850**

Deliver Bolus 1.5U

Voice Listener

FIG. 8

EP 4 401 084 A2

**900**

**910**
Smart Phone

Voice Listener

**915**
"Omnipod" 🔊))

**920**
Launch AID Menu

Voice Listener

**925**
"Quick Meal Bolus" 🔊))

**930**
Confirm Quick Meal Bolus?

Dose 3 U

"Yes" "No"

Voice Listener

**935**
"Yes" 🔊))

**940**
Deliver 3 U

Voice Listener

FIG. 9

1000

1010
Smart Phone
Voice Listener

1020
Launch AID Menu
Voice Listener

1030
Bolus for Meal
- Grilled Cheese Sandwich
- Coffee

"Yes" "No"

Voice Listener

1040
Bolusing for 30 g carb
Voice Listener

1050
Delivering 3U
Voice Listener

1015
"Omnipod"
◁))

1025
"Bolus for meal. Grilled cheese sandwich and coffee."
◁))

1035
"Yes"
◁))

FIG. 10

EP 4 401 084 A2

FIG. 11

**1200**

1210
| Smart Phone |
| Voice Listener |

1220
| Launch AID Menu |
| Voice Listener |

1230
| Confirm Activity Mode? |
| Duration 2 Hours |
| "Yes" | "No" |
| Voice Listener |

1240
| Set Activity Mode |
| Voice Listener |

1215
"Omnipod"

1225
"Set Activity Mode for 2 hours"

1235
"Yes"

FIG. 12

FIG. 13

FIG. 14

**1500**

FIG. 15

EP 4 401 084 A2

FIG. 16